(19) Europäisches Patentamt
European Patent Office
Office européen des brevets

(11) Veröffentlichungsnummer: **0 168 043**
**B1**

(12) # EUROPÄISCHE PATENTSCHRIFT

(45) Veröffentlichungstag der Patentschrift: **07.11.90**

(21) Anmeldenummer: **85108566.2**

(22) Anmeldetag: **10.07.85**

(51) Int. Cl.$^5$: **C 09 K 19/30**, C 09 K 19/34, C 09 K 19/20, C 07 D 211/00, C 07 D 239/00, C 07 D 319/00, C 07 D 339/00

(54) **Flüssigkristall-Phase.**

(30) Priorität: **11.07.84 DE 3425503**

(43) Veröffentlichungstag der Anmeldung: **15.01.86 Patentblatt 86/03**

(45) Bekanntmachung des Hinweises auf die Patenterteilung: **07.11.90 Patentblatt 90/45**

(84) Benannte Vertragsstaaten: **CH DE FR GB LI SE**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **Heppke, Gerd, Prof. Dr.**
**Johannn-Georg-Strasse 3**
**D-1000 Berlin 31 (DE)**
Erfinder: **Oestreicher, Feodor, Dr.**
**Zeppelingstrasse 40**
**D-1000 Berlin 20 (DE)**

(56) Entgegenhaltungen:
**EP-A-0 035 155**
**DE-A-3 137 058**

(56) Entgegenhaltungen:
**MOLECULAR CRYSTALS AND LIQUID CRYSTALS, Band 110, Nr. 1/4, 1984, Seiten 175-203, Gordon & Breach Science Publishers, New York, US; G.J. DIENES et al.: "Proceedings of the symposium on liquid crystals and ordered fluids, 9. - 11. April 1984"**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 168 043 B1

**Beschreibung**

Für Flüssigkristallanzeigen werden in zunehmenden Maße Flüssigkristall-Phasen benötigt, die eine Helixstruktur mit vorgegebenem Drehsinn ausbilden. So benötigt man derartige Materialien beispielsweise für den Schadt-Helfrich-Effekt, um den unerwünschten Effekt des "reverse twist" zu vermeiden (E. Guyon und W. Urbach in "Nonemissive Electrooptic Displays", Hrsg. A. R. Kmetz, F. K. von Willisen, Plenum Press, New York—London, 1976, Seite 127), für den chloesterisch-nematischen Phasenumwandlungseffekt sowie für Bistabilitätseffekte.

En wichtiges Problem ist dabei die Erzeugung einer geeigneten Temperaturfunktion der Helixganghöhe, die sich nach dem jeweiligen elektrooptischen Effekt und dessen spezieller Ausführung richtet.

Für Flüssigkristallanzeigeelemente auf der Basis der verdrillten nematischen Zelle kommt beispielsweise eine temperaturunabhängige Ganghöhe zur Vermeidung des "reverse twist" in Fragbe. Weiterhin konnte gezeigt werden, daß eine Kompensation der Temperaturdrift der Schwellenspannung einer verdrillten nematischen Zelle erreicht werden kann, wenn die Helixganghöhe mit zunehmender Temperatur abnimmt (P. R. Gerber, Physics Letters 78A, 285 (1980)). Gleichermaßen gilt für den Phasenumwandlungseffekt, daß durch stark abnehmende Helixganghöhe mit zunehmender Temperatur eine Kompensation der Schwellspannungsdrift erreicht wird (A. Göbl-Wunsch, G. Heppke und F. Oestreicher, Journal de Physique 40, 773 (1979)).

Im allgemeinen bestehen die für diese Zwecke angewendeten Flüssigkristall-Phasen aus Gemischen von nicht chiralen flüssigkristallinen Verbindungen, denen chirale Verbindungen zur Erzeugang der Helixstruktur zugesetzt werden. Praktisch alle bekannten chiralen Dotierstoffe induzieren Helixstrukturen, deren Ganghöhen über weite Bereiche mit der Temperatur mehr oder weniger stark zunehmen. In der Literatur wird lediglich über gewisse Spirobiindanderivate mit negativer Steigung der Temperaturfunktion berichtet (Advances of Infrared and Raman Spectroscopy *8* (1981) Kap. 4). In der Praxis ließ sich jedoch mit diesen Verbindungen die störende Temperaturdrift nicht beseitigen. Die oftmals gewünschte negative Steigung der Temperaturfunktion konnte bislang nur durch Verwendung zweier geeigneter Dotierstoffe unterschiedlichen Drehsinnes und unterschiedlicher relativer Temperaturabhängigkeit erreicht werden (DE—A—28 27 471). Nachteile dieses Mehrfachdotierungsverfahrens sind u.a. die Einhaltung des genauen Konzentrationsverhältnisses der beiden chiralen Verbindungen und die Einschränkung auf ein begrenztes Temperaturintervall sowie die notwendige hohe Gesamtkonzentration der Dotierstoffe (A. Göbl-Wunsch, G. Heppke und F. Oestreicher, Journal de Physique, *40,* 773 (1979)). Außenanwendungen sind daher nicht möglich.

Der Erfindung lag daher die Aufgabe zugrunde, eine Flüssigkristall-Phase aufzufinden, die über temperaturunabhängige elektrooptische Kenngrößen, insbesondere über eine temperaturunabhängige Schwellspannung, verfügt und diese Eigenschaften bereits mit Hilfe eines einzigen Dotierstoffes erhält.

Der Erfindung lag weiterhin die Aufgabe zugrunde, chirale Verbindungen zu finden, die eine negative Steigung der Temperaturfunktion der Ganghöhe in Flüssigkristall-Phasen für Innen- und Außenanwendungen über einen weiten Temperaturbereich induzieren bei gleichzeitig hohem Verdrillungsvermögen.

Diese Aufgaben werden erfindungsgemäß durch eine Flüssigkristall-Phase gemäß Anspruch 18 sowie durch die Bereitstellung der Verbindungen der Formel I gelöst.

Es wurde gefunden, daß die Verbindungen der Formel I den obigen Forderungen in vorzüglicher Weise nachkommen.

Gegenstand der Erfindung sind eine Flüssigkristall-Phase mit einem Gehalt an mindestens einer optisch aktiven Verbindung der Formel I

$$R^1—X^1—CHR^o—(CH_2)_p—X^2—R^2 \qquad I$$

worin $X^1$ und $X^2$ jeweils unabhängig voneinander —CO—O— oder —O—CO—, eine der Gruppen $X^1$ und $X^2$ auch —O—, wobei $R^1$ und $R^2$ unabhängig voneinander jeweils eine Gruppe $—(A^1—Z)_m—(A^2)_n—Y$ bedeuten, worin

$A^1$ und $A^2$ jeweils unabhängig voneinander eine 1,4-Phenylen-, Pyrimidin-2,5-diyl, Pyrazin-2,5-diyl, Pyridin-2,5-diyl, 1,4-Cyclohexylen-, 1,3-Dioxan-2,5,-diyl-, 1,3-Dithian-2,5-diyl- oder 1,4-Bicyclo(2,2,2)-octylen-Gruppe, wobei diese auch ein- oder mehrfach substituiert sein kann durch F, Cl, Br, Cn und/oder Alkylgruppen mit bis zu 12 C-Atomen, wobei in den Alkylgruppen 1 oder 2 nicht benachbarte $CH_2$-Gruppen durch O-Atome ersetzt sein können,

Z —CO—O—, —O—CO—, $—CH_2CH_2—$, $—OCH_2$, $—CH_2O—$, —CH=N—, —N=CH—, —N=N—, —N(O)=N— oder eine Einfachbindung,

m und n jeweils unabhängig voneinander 0, 1 oder 2 und

Y eine geradkettige oder verzweigte Alkylgruppe mit bis zu 12 C-Atomen, wobei 1 oder 2 nicht benachbarte und nicht endständige $CH_2$-Gruppen durch O-Atome ersetzt sein können, oder falls n 1 oder 2 ist, auch F, Cl, Br oder CN,

p 0 oder 1 ist, und

$R^o$ eine Alkylgruppe mit bis zu 5 C-Atomen, eine Phenylgruppe oder eine Cyclohexylgruppe ist.

Gegenstand der Erfindung sind ferner neue optisch aktive Verbindungen der Formel I

$$R^1—X^1—CHR^o—(CH_2)_p—X^2—R^2 \qquad I$$

worin $X^1$ und $X^2$ jeweils unabhängig voneinander —CO—O— oder —O—CO—, eine der Gruppen $X^1$ und $X^2$ auch —O—, wobei $R^1$ und $R^2$ unabhängig voneinander jeweils eine Gruppe $—(A^1—Z)_m—(A^2)_n—Y$ bedeuten, worin

$A^1$ und $A^2$ jeweils unhabhängig voneinander eine 1,4-Phenylen-, Pyrimidin-2,5-diyl, Pyrazin-2,5-diyl, Pyridin-2,5-diyl, 1,4-Cyclohexylen-, 1,3-Dioxan-2,5-diyl, 1,3-Dithian-2,5-diyl- oder 1,4-Bicyclo(2,2,2)-octylengruppe, wobei diese auch ein- oder mehrfach substituiert sein kann durch F, Cl, Br, CN und/oder Alkylgruppen mit bis zu 12 C-Atomen, wobei in den Alkylgruppen 1 oder 2 nicht benachbarte $CH_2$-Gruppen durch O-Atome ersetzt sein können,

Z —CO—O—, —O—CO—, $—CH_2CH_2—$, $—OCH_2$, $—CH_2O—$, —CH=N—, —N=CH—, —N=N—, —N(O)=N— oder eine Einfachbindung,

m und n jeweils unabhängig voneinander 0, 1 oder 2 und

Y eine geradkettige oder verzweigte Alkylgruppe mit bis zu 12 C-Atomen, wobei 1 oder 2 nicht benachbarte und nicht endständige $CH_2$-Gruppen durch O-Atome ersetzt zein können, oder falls n 1 oder 2 ist, auch F, Cl, Br oder CN, mit der Maßgabe, daß (m + n) in mindestens einer der Gruppen $R^1$ und $R^2$ 2, 3 oder 4 ist,

p 0 oder 1 ist, und

$R^o$ eine Alkylgruppe mit bis zu 5 C-Atomen, eine Phenylgruppe oder eine Cyclohexylgruppe ist.

Gegenstand der Erfindung ist weiterhin ein Flüssigkristallanzeigeelement, enthaltend eine erfindungsgemäße Flüssigkristall-Phase.

Weiterhin ist Gegenstand der Erfindung die Verwendung von Verbindungen der Formel I, zur Temperaturkompensation in Flüssigkristall-Phasen.

Gegenstand der Erfindung ist auch ein Verfahren zur Temperaturkompensation in Flüssigkristallanzeigeelementen, die eine Flüssigkristall-Phase enthalten, wobei man der Flüssigkristall-Phase mindestens 0,05% an mindestens einer chiralen Verbindung der Formel I zumischt.

Unter temperaturkompensierten Flüssigkristall-Phasen beziehungsweise Flüssigkristallanzeigeelementen sollen Flüssigkristall-Phasen beziehungsweise Flüssigkristallanzeigeelemente mit weitgehend temperaturunabhängigen elektrooptischen Kenngrößen, insbesondere mit weitgehend temperaturunabhängiger Schwellenspannung, verstanden werden.

Diese Temperaturunabhängigkeit ist im allgemeinen Teil für die normalen Anwendungsbereiche gewährleistet, d.h. von −40 bis +100°C, insbesondere von −20 bis +80°C. Eleketrooptische Kenngrößen werden hier, wie allgemein üblich, als weigehend temperaturunabhängig bezeichnet, falls diese im Temperaturbereich von 0 bis 40°C nicht mehr als ungefähr ± 0,4% pro Grad Celsius bzw. im Temperaturbereich von −20 bis +80°C nicht mehr als ungefähr ± 0,15% pro Grad Celsius, vorzugsweise nicht mehr als ungefähr ± 0,05% pro Grad Celsius, schwanken. Für den Fachmann liegt es auf der Hand, daß die Ausgeprägtheit oder erforderlichen Temperaturunabhängigkeit von der jeweils beabsichtigten Anwendung abhängt.

Die Verbindungen der Formel I umfassen neue optisch aktive Verbindungen Teilformeln I', I'', Ib', Ib'', Ic' und Ic'':

$$Y-X^1-CHR^o-(CH_2)_p-X^2-(A^1-Z)_m-(A^2)_n-Y \qquad I'$$

$$Y-(A^2)_n-(Z-A^1)_m-X^1-CHR^o-(CH_2)_p-X^2-Y \qquad I''$$

$$Y-X^1-CHR^o-(CH_2)_p-X^2-A^1-A^2-Y \qquad Ib'$$

$$Y-A^2-A^1-X^1-CHR^o-(CH_2)_p-X^2-Y \qquad Ib''$$

$$Y-X^1-CHR^o-(CH_2)_p-X^2-A^1-Z-A^2-Y \qquad Ic'$$

$$Y-A^2-Z-A^1-X^1-CHR^o-(CH_2)_p-X^2-Y \qquad Ic''$$

Unter den Verbindungen der Formeln I, I' und I'' sind diejenigen bevorzugt, worin $A^1$ und $A^2$ 1,4-Phenylen bedeuten oder worin eine der Gruppen $A^1$ und $A^2$ 1,4-Phenylen und die andere Gruppe Pyrimidin-2,5-diyl, Pyrazin-2,5-diyl oder Pyridin-2,5-diyl bedeutet.

Unter den Verbindungen der Formeln Ib' und Ib'' sind diejenigen bevorzugt, worin $—A^1—A^2—$

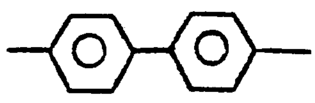, 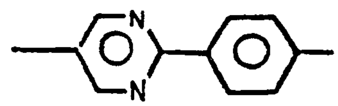,

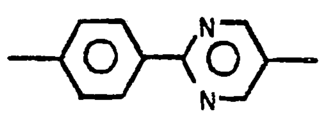, 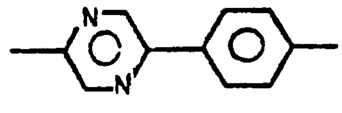,

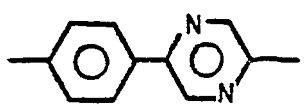, 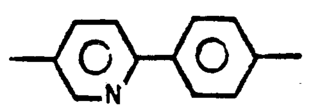 oder 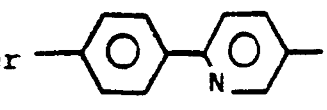

bedeutet.

Die neuen optisch aktiven Verbindungen der Formel I nach Anspruch 1 haben weiterhin eine Bedeutung als chirale Dotierstoffe für flüssigkristalline Mischungen.

Die Verbindungen der Formel I umfassen ferner bevorzugte Verbindungen der Teilformeln Ia bis Im:

| | |
|---|---|
| $Y-CO-O-CHR^{o}-CH_2-O-CO-A^1-Y$ | Ia |
| $Y-CO-O-CHR^{o}-CH_2-O-CO-A^1-A^2-Y$ | Ib |
| $Y-CO-O-CHR^{o}-CH_2-O-CO-A^1-Z-A^2-Y$ | Ic |
| $Y-CO-O-CHR^{o}-CH_2-O-CO-(A^1)_2-Z-A^2-Y$ | Id |
| $Y-CC-O-CHR^{o}-CH_2-O-CO-A^1-Z-(A^1)_2-Y$ | Ie |
| $Y-A^1-CO-O-CHR^{o}-CH_2-O-CO-A^1-Y$ | If |
| $Y-A^1-CO-O-CHR^{o}-CH_2-O-CO-A^1-A^2-Y$ | Ig |
| $Y-A^1-CO-O-CHR^{o}-CH_2-O-CO-A^1-Z-A^2-Y$ | Ih |
| $Y-A^1-CO-O-CHR^{o}-CH_2-O-CO-(A^1)_2-Z-A^2-Y$ | Ii |
| $Y-A^1-CO-O-CHR^{o}-CH_2-O-CO-A^1-Z-(A^2)_2-Y$ | Ij |
| $Y-A^1-A^2-CO-O-CHR^{o}-CH_2-O-CO-A^1-A^2-Y$ | Ik |
| $Y-A^1-A^2-CO-O-CHR^{o}-CH_2-O-CO-A^1-Z-A^2-Y$ | Il |
| $Y-A^2-Z-A^1-CO-O-CHR^{o}-CH_2-O-CO-A^1-Z-A^2-Y$ | Im |

In den Verbindungen der vor- und nachstehenden Formeln bedeutet Y vorzugsweise Alkyl, ferner Alkoxy, eine andere Oxaalkylgruppe, CN oder F.

In den vor- und nachstehenden Formeln bedeutet $R^{o}$ vorzugsweise Methyl oder Phenyl, p ist vorzugsweise 1, $X^1$ ist vorzugsweise —CO—O—, $X^2$ ist vorzugsweise —O—CO— oder —O— und Y ist vorzugsweise geradkettiges Alkyl mit bis zu 7 C-Atomen.

In den Verbindungen der vor- und nachstehenden Formeln können die Alkylreste, in denen auch eine ("Alkoxy" bzw. "Oxaalkyl") oder zwei $CH_2$-Gruppen ("Alkoxyalkoxy" bzw. "Dioxaalkyl") durch O-Atome ersetzt sein können, geradkettig oder verzweigt sein. Vorzugsweise sind sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2-Oxabutyl (= Ethoxymethyl) oder 3-Oxabutyl (= 2-Methoxyethyl), 2-, 3-, oder 4-Oxapentyl, 2-, 3-, 4- oder 5-Oxahexyl, 2-, 3-, 4-, 5- oder 6-Oxaheptyl, ferner Methyl, Octyl, Nonyl, Decyl, Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5- 2,6-, 3,5-, 3,6- oder 4,6-Dioxaheptyl.

Verbindungen der Formeln I sowie Ia bis Im mit verzweigten Flügelgruppen Y können auch von

Bedeutung sein. Verzweigte Gruppen dieser Art enthalten in der Regel nicht mehr als eine Kettenverzweigung. Bevorzugte verzweigte Reste Y sind Isopropyl, 2-Butyl (= 1-Methylpropyl), Isobutyl (= 2-Methylpropyl), 2-Methylbutyl, Isopentyl (= 3-Methylbutyl), 2-Methyl-pentyl, 3-Methyl-pentyl, 2-Ethylhexyl, 2-Propylpentyl, Isopropoxy, 2-Methylpropoxy, 2-Methylbutoxy, 3-Methyl-butoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Der Einfachheit halber bedeuten im folgenden "Phe" eine 1,4-Phenylengruppe, "Cy" eine 1,4-Cyclohexylengruppe, "Dio" eine 1,3-Dioxan-2,5-diylgruppe, "Bi" eine Bicyclo(2,2,2)-octylengruppe und "Pyr" eine Pyrimidin-2,5-diyl-gruppe, eine Pyrazin-2,5-diyl-Gruppe oder eine Pyridin-2,5-diyl-Gruppe, wobei diese Gruppen, unsubstituiert oder auch ein- oder mehrfach substituiert sein können durch F, Cl, Br, CN und/oder Alkylgruppen mit bis zu 12 C-Atomen, wobei in den Alkylgruppen 1 oder 2 $CH_2$-Gruppen durch O-Atome ersetzt sein können.

$A^1$ und $A^2$ sind jeweils unabhängig voneinander bevorzugt Cy, Phe oder Dio; bevorzugt enthält die Verbindung der Formel I nicht mehr als einen der Reste Dio, Bi oder Pyr.

Z ist bevorzugt eine Einfachbindung oder eine Carboxylgruppe. Z bedeutet ferner bevorzugt eine —$CH_2CH_2$—gruppe. (m + n) ist vorzugsweise 0, 1, 2 oder 3, besonders bevorzugt 0 oder 2.

Bevorzugte Gruppen $R_1$ und $R_2$ sind:

—Cy—Phe—Y
—Phe—Cy—Y
—Phe—Phe—Y
—Cy—Cy—Y
—Phe—Phe—Phe—Y
—Cy—Phe—Phe—Y
—Cy—Cy—Phe—Y
—Dio—Phe—Phe—Y
—Dio—Cy—Y
—Dio—Phe—Y
—Pyr—Phe—Y
—Pyr—Cy—Y
—$(A^1)_2$—$A^2$—Y
—$A^1$—$CH_2CH_2$—$A^2$—Y
—$A^1$—COO—$A^2$—Y
—$A^1$—OCO—$A^2$—Y
—Cy—Ph—$CH_2CH_2$—Cy—Y
—Cy—COO—Cy—Y
—Cy—COO—Ph—Y
—Ph—COO—Ph—Y

$R^o$ ist vorzugsweise eine geradkettige Alkylgruppe mit bis zu 5 C-Atomen oder eine Phenylgruppe, insbesondere bevorzugt eine Methyl- oder eine Phenylgruppe.

Pyr ist vorzugsweise eine Pyrimidin-2,5-diyl-Gruppe.

Die Verbindungen der Formel I enthalten vorzugsweise 0, 1, 2, 3, 4, 5, oder 6 Ringstrukturen $A^1$ und $A^2$. Besonders bevorzugt sind Verbindungen mit 2 bis 4 Ringstrukturen $A^1$ und $A^2$.

Unter den Verbindungen der Formeln I sowie Ia bis Im sind diejenigen bevorzugt, in denen mindestens einer der darin enthaltenen Reste eine der angegebenen bevorzugten Bedeutungen hat.

Die erfindungsgemäßen Flüssigkristall-Phasen bestehen aus 2 bis 18, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzonate, Cyclohexan-carbonsäurephenyl- oder cyclohexyl-ester, Phenylcyclohexane, Cyclohexylbiphenyle, Cyclohexylcyclohexane, Cyclohexylbiphenyle, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger Flüssigkristall-Phasen in Frage kommenden Verbindungen lassen sich durch die Formel III charakterisieren,

$$R^3—L—G—E—R^4 \qquad III$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituertem Napthalin, Di- und Tetrahydronapthalin, Chinazolin und Tetrahydrochinazolin gebildeten Gruppe,

| | | |
|---|---|---|
| G | $-CH=CH-$ | $-N(O)=N-$ |
| | $-CH=CQ-$ | $-CH=N(O)-$ |
| | $-C\equiv C-$ | $-CH_2-CH_2-$ |
| | $-CO-O-$ | $-CH_2-O-$ |
| | $-CO-S-$ | $-CH_2-S-$ |
| | $-CH=N-$ | $-COO-Phe-COO-$ |

oder eine C—C—Einfachbindung, Q Halogen, vorzugsweise Chlor, oder —CN, und $R^3$ und $R^4$ jeweils Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^3$ und $R^4$ voneinander verschieden, wobei einer dieser Reste meist eine Alkyl- oder Alkoxygruppe ist. Auch andere Varianten der vorgesehenen Substituenten sind gebräuchlich. Viele solcher Substanzen oder auch Gemische davon sind im Handel erhältlich. Alle diese Substanzen sind nach literaturebekannten Methoden herstellbar.

Die erfindungsgemäßen Flüssigkristall-Phasen enthalten mindestens 0,05% an mindestens einer Verbindung der Formel I. Vorzugsweise enthalten sie etwa 0,05 bis 35%, insbesonder 0,1 bis 10%, einer oder mehrerer Verbindungen der Formel I.

Für flüssigkristalline Dielektrika besonders bevorzugte erfindungsgemäße Flüssigkristall-Phasen enthalten 0,1 bis 3% einer oder mehrerer Verbindungen der Formel I. Besonders bevorzugt sind Dielektrika, die nur eine Verbindung der Formel I enthalten.

Die Herstellung der erfindungsgemäßen Flüssigkristall-Phasen erfolgt in an sich üblicher Weise. In der Regel werden die Komponenten ineinander gelöst, zweckmäßig bei erhöhter Temperatur. Durch geeignete Zusätze können die Flüssigkristall-Phasen nach der Erfindung so modifiziert werden, daß sie in allen bisher bekannt gewordenen Arten von Flüssigkristallanzeigeelementen verwendet werden können.

Die Verbindungen der Formel I können weiterhin, gegebenenfalls auch ohne Zumischung weiterer Komponenten, als Flüssigkristall-Phasen für Temperaturindikatoren verwendet werden.

In den hierfür in Frage kommenden Verbindungen der Formel I enthält mindestens eine der Gruppen $R^1$ oder $R^2$ zwei Ringstrukturen.

Die obengenannten Zusätze sind dem Fachmann bekannt und in der Literatur ausführlich beschrieben. Beispielsweise können Leitsalze, vorzgusweise Ethyl-dimethy-dodecyl-ammonium-4-hexyloxybenzoat, Tetrabutylammonium-tetraphenylboranat oder Komplexsalze von Kronenethern (vgl. z.B. I. Haller et al., Mol Cryst. Liq. Cryst. Band *24*, Seiten 249—258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind zum Beispiel in den DE—OS 22 09 127, 22 40 864, 23 21 632, 23 38 281, 24 50 088, 25 37 430, 28 53 728 und 29 02 177 beschrieben.

Alle erfindungsgemäßen chiralen Verbindungen der Formel I sind bekannt oder in einfacher Weise nach konventionellen Synthesverfahren aus bekannten Ausgangsmaterialen herstellbar. Die neuen Verbindungen können in analoger Weise wie die bekannten Verbindungen hergestellt werden (Houben-Weyl, Methoden der Organischen Chemie, Georg-Thieme Verlag, Stuttgart; Organic Reactions, John Wiley & Sons, Inc., New York; Morrison and Mosher, Asymmetric Organic Reactions, Prentice-Hall, Inc., Englewood Cliffs, N.Y., 1971; Wilen, Top. Stereochem, *6*, 107—176 (1971); March, Advanced Organic Chemistry, McGraw-Hill series in Advanced Chemistry, McGraw-Hill Kogakusha, Tokyo, 1977).

Vorzugsweise werden die Verbindungen der Formel I durch Veresterung entsprechender Carbonsäuren (oder deren reaktionsfähiger Derivate) mit Alkoholen oder Phenolen (oder deren reaktionsfähigen Derivaten) hergestellt.

Als reaktionsfähige Derivate der genannten Carbonsäuren eigen sich insbesondere die Säurehalogenide, vor allem die Chloride und Bromide, ferner die Anhydride, Azide oder Ester, insbesondere Alkylester mit 1—4 C-Atomen in der Alkylgruppe.

Als reaktionsfähige Derivate der genannten Alkohole bzw. Phenole kommen insbesondere die entsprechenden Metallalkoholate bzw. -phenolate in Betracht, vor allem die Natrium- und Kaliumalkoholate bzw. -phenolate.

Die Veresterung wird vorteilhaft in Gegenwart eines inerten Lösungsmittels durchgeführt. Gut geeignet sind insbesondere Ether wie Diethylether, Di-n-butylether, Tetrahydrofuran, Dioxan oder Anisol, Ketone wie Aceton, Butanon oder Cyclohexanon, Amide wie Dimethylformamid oder Phosphorsäurehexamethyltriamid, Kohlenwasserstoffe wie Benzol, Toluol oder Xylol, Halogen-kohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer

organischen Base, z.B. Pyridin, Chinolin oder Triethylamin als Lösungsmittel für die Veresterung angewandt werden.

Die Veresterung kann auch in Abwesenheit eines Lösungsmittels, zum Beispiel durch einfaches Erhitzen der Komponenten in Gegenwart von Natriumacetat, durchgeführt werden. Die Reaktionstemperatur ist normalerweise −50 bis +250°C, vorzugsweise −20 bis +80°C. Bei diesen Temperaturen sind die Veresterungen in der Regel nach 15 Minuten bis 48 Studen beendet.

Im einzelnen hängen die Reaktionsbedingungen für die Veresterung weitgehend von der Natur der verwendeten Ausgangsstoffe ab. So wird eine freie Carbonsäure mit einen freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid-, Alkalimetallcarbonate bzw. -hydrogencarbonate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kaliauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa −25° und +20°C.

Die folgenden Beispiele zeigen typsiche konventionelle Syntheseverfahren, die zur Herstellung erfindungsgemäßer Verbindungen geeignet sind und sollen die Erfindung erläutern, ohne sie zu begrenzen. F. = Schmelzpunkt.

Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt ab. trocknet die organische phase, dampft ein und reinigt das produkt durch Kristallisation und/oder Chromatographie.

Beispiel 1

6,4 g p-(trans-4-n-Heptylcyclohexyl)-benzoesäurechlorid [erhältlich durch Umsetzung von p-(trans-4-n-Heptylcyclohexyl)-benzoesäure mit Thionylchlorid] in 20 ml Toluol werden zu einem Gemisch aus 0,8 g (S)-1,2-Propandiol, 20 ml Toluol und 2,4 ml Pyridin bei 50° zugetropft. Das Reaktionsgemisch wird 8 Stunden gekocht. Das Pyridiniumchlorid wird heiß abgesaugt und das Filtrat wie üblich aufgearbeitet. Man erhält optisch aktives 1,2-Bis-(p-trans-4-n-heptylcyclohexylbenzoyloxy)-propan, F. 97°.

Analog werden hergestellt:

1,2-Bis-(p-trans-4-ethylcyclohexylbenzoyloxy)-propan
1,2-Bis-(p-trans-4-propylcyclohexylbenzoyloxy)-propan
1,2-Bis-(p-trans-4-butylcyclohexylbenzoyloxy)-propan
1,2-Bis-(p-trans-4-pentylcyclohexylbenzoyloxy)-propan
1,2-Bis-(p-trans-4-hexylcyclohexylbenzoyloxy)-propan
1,2-Bis-[p-(p'-trans-4-ethylcyclohexylphenyl)-benzoyloxy]-propan
1,2-Bis-[p-(p'-trans-4-propylcyclohexylphenyl)-benzoyloxy]-propan
1,2-Bis-[p-(p'-trans-4-butylcyclohexylphenyl)-benzoyloxy]-propan
1,2-Bis-[p-(p'-trans-4-pentylcyclohexylphenyl)-benzoyloxy]-propan
1,2-Bis-[p-(p'-trans-4-hexylcyclohexylphenyl)-benzoyloxy]-propan
1,2-Bis-[p-(p'-trans-4-heptylcyclohexylphenyl)-benzoyloxy]-propan
1,2-Bis-[p-(p'-trans-4-octylcyclohexylphenyl)-benzoyloxy]-propan
1,2-Bis-[p-(p'-trans-4-decylcyclohexylphenyl)-benzoyloxy]-propan
1,2-Bis-(trans,trans-4-ethylcyclohexylcyclohexyl-4'-carbonyloxy)-propan
1,2-Bis-(trans,trans-4-propylcyclohexylcyclohexyl-4'-carbonyloxy)-propan
1,2-Bis-(trans,trans-4-butylcyclohexylcyclohexyl-4'-carbonyloxy)-propan
1,2-Bis-(trans,trans-4-pentylcyclohexylcyclohexyl-4'-carbonyloxy)-propan
1,2-Bis-(trans,trans-4-hexylcyclohexylcyclohexyl-4'-carbonyloxy)-propan
1,2-Bis-(trans,trans-4-heptylcyclohexylcyclohexyl-4'-carbonyloxy)-propan
1,2-Bis-(trans,trans-4-octylcyclohexylcyclohexyl-4'-carbonyloxy)-propan
1,2-Bis-(trans,trans-4-decylcyclohexylcyclohexyl-4'-carbonyloxy)-propan
1,2-Bis-[p-(p'-ethoxybenzoyloxy)-benzoyloxy]-propan
1,2-Bis-[p-(p'-propoxybenzoyloxy)-benzoyloxy]-propan
1,2-Bis-[p-(p'-butoxybenzoyloxy)-benzoyloxy]-propan
1,2-Bis-[p-(p'-pentoxybenzoyloxy)-benzoyloxy]-propan
1,2-Bis-[p-(p'-hexoxybenzoyloxy)-benzoyloxy]-propan

133°

1,2-Bis-[p-(p'-heptoxybenzoyloxy)-benzoyloxy]-propan
1,2-Bis-[p-(p'-octoxybenzoyloxy)-benzoyloxy]-propan

1,2-Bis-[p-(p'-decoxybenzoyloxy)-benzoyloxy]-propan
1,2-Bis-[p-(p'-cyanbenzoyloxy)-benzoyloxy]-propan
1,2-Bis-[p-(p'-ethylbenzoyloxy)-benzoyloxy]-propan
1,2-Bis-[p-(p'-butylbenzoyloxy)-benzoyloxy]-propan
1,2-Bis-[p-(p'-pentylbenzoyloxy)-benzoyloxy]-propan
1,2-Bis-[p-(p'-heptylbenzoyloxy)-benzoyloxy]-propan
1,2-Bis-(p-ethoxybenzoyloxy)-propan
1,2-Bis-(p-propoxybenzoyloxy)-propan
1,2-Bis-(p-butoxybenzoyloxy)-propan
1,2-Bis-(p-pentoxybenzoyloxy)-propan
1,2-Bis-(p-hexoxybenzoyloxy)-propan
1,2-Bis-(p-heptoxybenzoyloxy)-propan
1,2-Bis-(p-octoxybenzoyloxy)-propan
1,2-Bis-(p-decoxybenzoyloxy)-propan
1,2-Bis-(p-ethylbenzoyloxy)-propan
1,2-Bis-(p-propylbenzoyloxy)-propan
1,2-Bis-(p-butylbenzoyloxy)-propan
1,2-Bis-(p-pentylbenzoyloxy)-propan
1,2-Bis-(p-hexylbenzoyloxy)-propan
1,2-Bis-(p-heptylbenzoyloxy)-propan
1,2-Bis-(p-octylbenzoyloxy)-propan
1,2-Bis-(p-decylbenzoyloxy)-propan
1,2-Bis-(p-1-methylpropylbenzoyloxy)-propan
1,2-Bis-(p-(R)-2-methylpropylbenzoyloxy)-propan
1,2-Bis-(p-(R)-2-methylbutylbenzoyloxy)-propan
1,2-Bis-(p-(S)-3-methylbutylbenzoyloxy)-propan
1,2-Bis-(p-(R)-2-methylpentylbenzoyloxy)-propan
1,2-Bis-(p-2-ethylhexylbenzoyloxy)-propan
1,2-Bis-(p-(S)-2-methylpropoxybenzoyloxy)-propan
1,2-Bis-(p-(R)-2-methylbutoxybenzoyloxy)-propan
1,2-Bis-(p-(S)-3-methylbutoxybenzoyloxy)-propan
1,2-Bis-(p-(R)-2-methylpentoxybenzoyloxy)-propan
1,2-Bis-(p-2-ethylhexoxybenzoyloxy)-propan
1,2-Bis-(p-(R)-2-oxa-3-methylbutylbenzoyloxy)-propan

Beispeil 2

6,4 g p-(trans-4-n-Heptylcyclohexyl)-benzosäurechlorid in 20 ml Toluol werden entsprechend Beispiel 1 mit einem Gemisch aus 1,4 g (R)-Phenyl-1,2-ethandiol, 20 ml Toluol und 2,4 ml Pyridin Umgesetz. Man erhält optisch aktives 1,2-Bis-(p-trans-4-n-heptyl-cyclohexylbenzoyloxy)-1-phenylethan, F. 124°.

Analog werden hergestellt:

1,2-Bis-(p-trans-4-ethylcyclohexylbenzoyloxy)-1-phenylethan
1,2-Bis-(p-trans-4-propylcyclohexylbenzoyloxy)-1-phenylethan
1,2-Bis-(p-trans-4-butylcyclohexylbenzoyloxy)-1-phenylethan
1,2-Bis-(p-trans-4-pentylcyclohexylbenzoyloxy)-1-phenylethan
1,2-Bis-(p-trans-4-hexylcyclohexylbenzoyloxy)-1-phenylethan
1,2-Bis-[p-(p'-trans-4-ethylcyclohexylphenyl)-benzoyloxy]-1-phenylethan
1,2-Bis-[p-(p'-trans-4-propylcyclohexylphenyl)-benzoyloxy]-1-phenylethan
1,2-Bis-[p-(p'-trans-4-butylcyclohexylphenyl)-benzoyloxy]-1-phenylethan
1,2-Bis-[p-(p'-trans-4-pentylcyclohexylphenyl)-benzoyloxy]-1-phenylethan
1,2-Bis-[p-(p'-trans-4-hexylcyclohexylphenyl)-benzoyloxy]-1-phenylethan
1,2-Bis-[p-(p'-trans-4-heptylcyclohexylphenyl)-benzoyloxy]-1-phenylethan
1,2-Bis-[p-(p'-trans-4-octylcyclohexylphenyl)-benzoyloxy]-1-phenylethan
1,2-Bis-[p-(p'-trans-4-decylcyclohexylphenyl)-benzoyloxy]-1-phenylethan
1,2-Bis-(trans,trans-4-ethylcyclohexylcyclohexyl-4'-carbonyloxy)-1-phenylethan
1,2-Bis-(trans,trans-4-propylcyclohexylcyclohexyl-4'-carbonyloxy)-1-phenylethan
1,2-Bis-(trans,trans-4-butylcyclohexylcyclohexyl-4'-carbonyloxy)-1-phenylethan
1,2-Bis-(trans,trans-4-pentylcyclohexylcyclohexyl-4'-carbonyloxy)-1-phenylethan
1,2-Bis-(trans,trans-4-hexylcyclohexylcyclohexyl-4'-carbonyloxy)-1-phenylethan
1,2-Bis-(trans,trans-4-heptylcyclohexylcyclohexyl-4'-carbonyloxy)-1-phenylethan
1,2-Bis-(trans,trans-4-octylcyclohexylcyclohexyl-4'-carbonyloxy)-1-phenylethan
1,2-Bis-(trans,trans-4-decylcyclohexylcyclohexyl-4'-carbonyloxy)-1-phenylethan
1,2-Bis-[p-(p'-ethoxybenzoyloxy)-benzoyloxy]-1-phenylethan
1,2-Bis-[p-(p'-propoxybenzoyloxy)-benzoyloxy]-1-phenylethan
1,2-Bis-[p-(p'-butoxybenzoyloxy)-benzoyloxy]-1-phenylethan

1,2-Bis-[p-(p'-pentoxybenzoyloxy)-benzoyloxy]-1-phenylethan
1,2-Bis-[p-(p'-hexoxybenzoyloxy)-benzoyloxy]-1-phenylethan

133°

1,2-Bis-[p-(p'-heptoxybenzoyloxy)-benzoyloxy]-1-phenylethan
1,2-Bis-[p-(p'-octoxybenzoyloxy)-benzoyloxy]-1-phenylethan
1,2-Bis-[p-(p'-decoxybenzoyloxy)-benzoyloxy]-1-phenylethan
1,2-Bis-[p-(p'-cyanbenzoyloxy)-benzoyloxy]-1-phenylethan
1,2-Bis-[p-(p'-ethylbenzoyloxy)-benzoyloxy]-1-phenylethan
1,2-Bis-[p-(p'-butylbenzoyloxy)-benzoyloxy]-1-phenylethan
1,2-Bis-[p-(p'-pentylbenzoyloxy)-benzoyloxy]-1-phenylethan
1,2-Bis-[p-(p'-heptylbenzoyloxy)-benzoyloxy]-1-phenylethan
1,2-Bis-(p-ethoxybenzoyloxy)-1-phenylethan
1,2-Bis-(p-propoxybenzoyloxy)-1-phenylethan
1,2-Bis-(p-butoxybenzoyloxy)-1-phenylethan
1,2-Bis-(p-pentoxybenzoyloxy)-1-phenylethan
1,2-Bis-(p-hexoxybenzoyloxy)-1-phenylethan
1,2-Bis-(p-heptoxybenzoyloxy)-1-phenylethan
1,2-Bis-(p-octoxybenzoyloxy)-1-phenylethan
1,2-Bis-(p-decoxybenzoyloxy)-1-phenylethan
1,2-Bis-(p-ethylbenzoyloxy)-1-phenylethan
1,2-Bis-(p-propylbenzoyloxy)-1-phenylethan
1,2-Bis-(p-butylbenzoyloxy)-1-phenylethan
1,2-Bis-(p-pentylbenzoyloxy)-1-phenylethan
1,2-Bis-(p-hexylbenzoyloxy)-1-phenylethan
1,2-Bis-(p-heptylbenzoyloxy)-1-phenylethan
1,2-Bis-(p-octylbenzoyloxy)-1-phenylethan
1,2-Bis-(p-decylbenzoyloxy)-1-phenylethan
1,2-Bis-(p-1-methylpropylbenzoyloxy)-1-phenylethan
1,2-Bis-(p-(R)-2-methylpropylbenzoyloxy)-1-phenylethan
1,2-Bis-(p-(R)-2-methylbutylbenzoyloxy)-1-phenylethan
1,2-Bis-(p-(S)-3-methylbutylbenzoyloxy)-1-phenylethan
1,2-Bis-(p-(R)-2-methylpentylbenzoyloxy)-1-phenylethan
1,2-Bis-(p-2-ethylhexylbenzoyloxy)-1-phenylethan
1,2-Bis-(p-(S)-2-methylpropoxybenzoyloxy)-1-phenylethan
1,2-Bis-(p-(R)-2-methylbutoxybenzoyloxy)-1-phenylethan
1,2-Bis-(p-(S)-3-methylbutoxybenzoyloxy)-1-phenylethan
1,2-Bis-(p-(R)-2-methylpentoxybenzoyloxy)-1-phenylethan
1,2-Bis-(p-2-ethylhexoxybenzoyloxy)-1-phenylethan
1,2-Bis-(p-(R)-2-oxa-3-methylbutylbenzoyloxy)-1-phenylethan.

## Beispiel 3

20 g p-(trans-4n-Heptylcyclohexyl)-benzoesäurechlorid in 50 ml Toluol werden entsprechend Beispiel 1 mit einem Gemisch aus 50 g (R)-Cyclohexyl-1,2-ethandiol, 50 ml Toluol und 8 ml Pyridin umgesetzt. Man erhält optisch aktives 1,2-Bis-(p-trans-4-n-heptylcyclohexyl-benzoyloxy)-1-cyclohexylethan.

Analog werden hergestellt:

1,2-Bis-(p-trans-4-ethylcyclohexylbenzoyloxy)-1-cyclohexylethan
1,2-Bis-(p-trans-4-propylcyclohexylbenzoyloxy)-1-cyclohexylethan
1,2-Bis-(p-trans-4-butylcyclohexylbenzoyloxy)-1-cyclohexylethan
1,2-Bis-(p-trans-4-pentylcyclohexylbenzoyloxy)-1-cyclohexylethan
1,2-Bis-(p-trans-4-hexylcyclohexylbenzoyloxy)-1-cyclohexylethan
1,2-Bis-[p-(p'-trans-4-ethylcyclohexylphenyl)-benzoyloxy]-1-cyclohexylethan
1,2-Bis-[p-(p'-trans-4-propylcyclohexylphenyl)-benzoyloxy]-1-cyclohexylethan
1,2-Bis-[p-(p'-trans-4-butylcyclohexylphenyl)-benzoyloxy]-1-cyclohexylethan
1,2-Bis-[p-(p'-trans-4-pentylcyclohexylphenyl)-benzoyloxy]-1-cyclohexylethan
1,2-Bis-[p-(p'-trans-4-hexylcyclohexylphenyl)-benzoyloxy]-1-cyclohexylethan
1,2-Bis-[p-(p'-trans-4-heptylcyclohexylphenyl)-benzoyloxy]-1-cyclohexylethan
1,2-Bis-[p-(p'-trans-4-octylcyclohexylphenyl)-benzoyloxy]-1-cyclohexylethan
1,2-Bis-[p-(p'-trans-4-decylcyclohexylphenyl)-benzoyloxy]-1-cyclohexylethan
1,2-Bis-(trans,trans-4-ethylcyclohexylcyclohexyl-4'-carbonyloxy)-1-cyclohexylethan
1,2-Bis-(trans,trans-4-propylcyclohexylcyclohexyl-4'-carbonyloxy)-1-cyclohexylethan
1,2-Bis-(trans,trans-4-butylcyclohexylcyclohexyl-4'-carbonyloxy)-1-cyclohexylethan
1,2-Bis-(trans,trans-4-pentylcyclohexylcyclohexyl-4'-carbonyloxy)-1-cyclohexylethan
1,2-Bis-(trans,trans-4-hexylcyclohexylcyclohexyl-4'-carbonyloxy)-1-cyclohexylethan
1,2-Bis-(trans,trans-4-heptylcyclohexylcyclohexyl-4'-carbonyloxy)-1-cyclohexylethan

1,2-Bis-(trans,trans-4-octylcyclohexylcyclohexyl-4'-carbonyloxy)-1-cyclohexylethan
1,2-Bis-(trans,trans-4-decylcyclohexylcyclohexyl-4'-carbonyloxy)-1-cyclohexylethan
1,2-Bis-[p-(p'-ethoxybenzoyloxy)-benzoyloxy]-1-cyclohexylethan
1,2-Bis-[p-(p'-propoxybenzoyloxy)-benzoyloxy]-1-cyclohexylethan
1,2-Bis-[p-(p'-butoxybenzoyloxy)-benzoyloxy]-1-cyclohexylethan
1,2-Bis-[p-(p'-pentoxybenzoyloxy)-benzoyloxy]-1-cyclohexylethan
1,2-Bis-[p-(p'-hexoxybenzoyloxy)-benzoyloxy]-1-cyclohexylethan

133°

1,2-Bis-[p-(p'-heptoxybenzoyloxy)-benzoyloxy]-1-cyclohexylethan
1,2-Bis-[p-(p'-octoxybenzoyloxy)-benzoyloxy]-1-cyclohexylethan
1,2-Bis-[p-(p'-decoxybenzoyloxy)-benzoyloxy]-1-cyclohexylethan
1,2-Bis-[p-(p'-cyanbenzoyloxy)-benzoyloxy]-1-cyclohexylethan
1,2-Bis-[p-(p'-ethylbenzoyloxy)-benzoyloxy]-1-cyclohexylethan
1,2-Bis-[p-(p'-butylbenzoyloxy)-benzoyloxy]-1-cyclohexylethan
1,2-Bis-[p-(p'-pentylbenzoyloxy)-benzoyloxy]-1-cyclohexylethan
1,2-Bis-[p-(p'-heptylbenzoyloxy)-benzoyloxy]-1-cyclohexylethan
1,2-Bis-(p-ethoxybenzoyloxy)-1-cyclohexylethan
1,2-Bis-(p-propoxybenzoyloxy)-1-cyclohexylethan
1,2-Bis-(p-butoxybenzoyloxy)-1-cyclohexylethan
1,2-Bis-(p-pentoxybenzoyloxy)-1-cyclohexylethan
1,2-Bis-(p-hexoxybenzoyloxy)-1-cyclohexylethan
1,2-Bis-(p-heptoxybenzoyloxy)-1-cyclohexylethan
1,2-Bis-(p-octoxybenzoyloxy)-1-cyclohexylethan
1,2-Bis-(p-decoxybenzoyloxy)-1-cyclohexylethan
1,2-Bis-(p-ethylbenzoyloxy)-1-cyclohexylethan
1,2-Bis-(p-propylbenzoyloxy)-1-cyclohexylethan
1,2-Bis-(p-butylbenzoyloxy)-1-cyclohexylethan
1,2-Bis-(p-pentylbenzoyloxy)-1-cyclohexylethan
1,2-Bis-(p-hexylbenzoyloxy)-1-cyclohexylethan
1,2-Bis-(p-heptylbenzoyloxy)-1-cyclohexylethan
1,2-Bis-(p-octylbenzoyloxy)-1-cyclohexylethan
1,2-Bis-(p-decylbenzoyloxy)-1-cyclohexylethan
1,2-Bis-(p-1-methylpropylbenzoyloxy)-1-cyclohexylethan
1,2-Bis-(p-(R)-2-methylpropylbenzoyloxy)-1-cyclohexylethan
1,2-Bis-(p-(R)-2-methylbutylbenzoyloxy)-1-cyclohexylethan
1,2-Bis-(p-(S)-3-methylbutylbenzoyloxy)-1-cyclohexylethan
1,2-Bis-(p-(R)-2-methylpentylbenzoyloxy)-1-cyclohexylethan
1,2-Bis-(p-2-ethylhexylbenzoyloxy)-1-cyclohexylethan
1,2-Bis-(p-(S)-2-methylpropoxybenzoyloxy)-1-cyclohexylethan
1,2-Bis-(p-(R)-2-methylbutoxybenzoyloxy)-1-cyclohexylethan
1,2-Bis-(p-(S)-3-methylbutoxybenzoyloxy)-1-cyclohexylethan
1,2-Bis-(p-(R)-2-methylpentoxybenzoyloxy)-1-cyclohexylethan
1,2-Bis-(p-2-ethylhexoxybenzoyloxy)-1-cyclohexylethan
1,2-Bis-(p-(R)-2-oxa-3-methylbutylbenzoyloxy)-1-cyclohexylethan

## Beispiel 4

Ein Gemisch von 0,4 g optisch aktivem 1-Methoxy-2-propanol, 1,7 g p-(p-n-Octylphenyl)-benzosäurechlorid, 20 ml Toluol und 2,1 ml Pyridin wird 6 Std. am Rückfluß gekocht. Das Pyridinhydrochlorid wird heiß abgesaugt und das Filtrat wie üblich aufgearbeitet. Man erhält optisch aktives 1-Methoxy-2-[p-(p-n-octylphenyl)-benzoyloxy]-propan.

Die folgenden optisch aktiven Verbindungen werden analog hergestellt:

1-Methoxy-2-[p-(p-n-propylphenyl)-benzoyloxy]-propan
1-Methoxy-2-[p-(p-n-pentylphenyl)-benzoyloxy]-propan
1-Methoxy-2-[p-(p-n-hexylphenyl)-benzoyloxy]-propan
1-Methoxy-2-[p-(p-n-heptyphenyl)-benzoyloxy]-propan
1-Methoxy-2-[p-(p-n-nonylphenyl)-benzoyloxy]-propan
1-Methoxy-2-[p-(p-n-decylphenyl)-benzoyloxy]-propan
1-Methoxy-2-[p-(p-n-butoxyphenyl)-benzoyloxy]-propan
1-Methoxy-2-[p-(p-n-hexoxyphenyl)-benzoyloxy]-propan
1-Methoxy-2-[p-(p-n-heptoxyphenyl)-benzoyloxy]-propan
1-Methoxy-2-[p-(p-n-octoxyphenyl)-benzoyloxy]-propan
1-Methoxy-2-[p-(p-n-nonoxyphenyl)-benzoyloxy]-propan
1-Ethoxy-2-[p-(p-propylphenyl)-benzoyloxy]-propan
1-Ethoxy-2-[p-(p-pentylphenyl)-benzoyloxy]-propan

1-Ethoxy-2-[p-(p-hexylphenyl)-benzoyloxy]-propan
1-Ethoxy-2-[p-(p-heptylphenyl)-benzoyloxy]-propan
1-Ethoxy-2-[p-(p-octylphenyl)-benzoyloxy]-propan
1-Ethoxy-2-[p-(p-nonylphenyl)-benzoyloxy]-propan
1-Ethoxy-2-[p-(p-decylphenyl)-benzoyloxy]-propan
1-Ethoxy-2-[p-(p-butoxyphenyl)-benzoyloxy]-propan
1-Ethoxy-2-[p-(p-pentoxyphenyl)-benzoyloxy]-propan
1-Ethoxy-2-[p-(p-hexoxyphenyl)-benzoyloxy]-propan
1-Ethoxy-2-[p-(p-heptoxyphenyl)-benzoyloxy]-propan
1-Ethoxy-2-[p-(p-octoxyphenyl)-benzoyloxy]-propan
1-Ethoxy-2-[p-(p-nonoxyphenyl)-benzoyloxy]-propan
1-Propoxy-2-[p-(p-propylphenyl)-benzoyloxy]-propan
1-Propoxy-2-[p-(p-pentylphenyl)-benzoyloxy]-propan
1-Propoxy-2-[p-(p-hexylphenyl)-benzoyloxy]-propan
1-Propoxy-2-[p-(p-heptylphenyl)-benzoyloxy]-propan
1-Propoxy-2-[p-(p-octylphenyl)-benzoyloxy]-propan
1-Propoxy-2-[p-(p-nonylphenyl)-benzoyloxy]-propan
1-Propoxy-2-[p-(p-decylphenyl)-benzoyloxy]-propan
1-Propoxy-2-[p-(p-butoxyphenyl)-benzoyloxy]-propan
1-Propoxy-2-[p-(p-pentoxyphenyl)-benzoyloxy]-propan
1-Propoxy-2-[p-(p-hexoxyphenyl)-benzoyloxy]-propan
1-Propoxy-2-[p-(p-heptoxyphenyl)-benzoyloxy]-propan
1-Propoxy-2-[p-(p-octoxyphenyl)-benzoyloxy]-propan
1-Propoxy-2-[p-(p-nonoxyphenyl)-benzoyloxy]-propan
1-Butoxy-2-[p-(p-propylphenyl)-benzoyloxy]-propan
1-Butoxy-2-[p-(p-pentylphenyl)-benzoyloxy]-propan
1-Butoxy-2-[p-(p-hexylphenyl)-benzoyloxy]-propan
1-Butoxy-2-[p-(p-heptylphenyl)-benzoyloxy]-propan
1-Butoxy-2-[p-(p-octylphenyl)-benzoyloxy]-propan
1-Butoxy-2-[p-(p-nonylphenyl)-benzoyloxy]-propan
1-Butoxy-2-[p-(p-decylphenyl)-benzoyloxy]-propan
1-Butoxy-2-[p-(p-butoxyphenyl)-benzoyloxy]-propan
1-Butoxy-2-[p-(p-pentoxyphenyl)-benzoyloxy]-propan
1-Butoxy-2-[p-(p-hexoxyphenyl)-benzoyloxy]-propan
1-Butoxy-2-[p-(p-heptoxyphenyl)-benzoyloxy]-propan
1-Butoxy-2-[p-(p-octoxyphenyl)-benzoyloxy]-propan
1-Butoxy-2-[p-(p-nonoxyphenyl)-benzoyloxy]-propan

## Beispiel 5

Eine Lösung von 11,6 g Dicyclohexylcarbodiimid in 6 ml Dichlormethan wird langsam bei 0° zu einer Lösung von 15,2 g 4-(5-n-Nonylpyrimidinyl-2-)-phenol, 5,2 g optisch aktive 2-Methyl-3-propoxy-propionsäure und 0,62 g 4-N,N-Dimethylaminopyridin in 100 ml Dichlormethan getropft. Nach Erwärmen auf Raumtemperatur wird zwei Stunden gerührt, vom Harnstoffderivat abfiltriert, das Filtrat mit verdünnter Salzsäure und Wasser gewaschen und die organische Phase wie üblich aufgearbeitet. Man erhält optisch aktives 4-(5-n-Nonyl-pyrimidinyl-2)-phenyl-2-methyl-3-propoxypropionat.

Die folgenden optisch aktiven Verbindungen werden analog hergestellt:

4-(5-Octylpyrimidinyl-2)-phenyl-2-methyl-3-propoxypropionat
4-(5-Heptylpyrimidinyl-2)-phenyl-2-methyl-3-propoxypropionat
4-(5-Hexylpyrimidinyl-2)-phenyl-2-methyl-3-propoxypropionat
4-(5-Pentylpyrimidinyl-2)-phenyl-2-methyl-3-propoxypropionat
4-(5-Butylpyrimidinyl-2)-phenyl-2-methyl-3-propoxypropionat
4-(5-Propylpyrimidinyl-2)-phenyl-2-methyl-3-propoxypropionat
4-(5-Nonylpyrimidinyl-2)-phenyl-2-methyl-3-methoxypropionat
4-(5-Octylpyrimidinyl-2)-phenyl-2-methyl-3-methoxypropionat
4-(5-Heptylpyrimidinyl-2)-phenyl-2-methyl-3-methoxypropionat
4-(5-Hexylpyrimidinyl-2)-phenyl-2-methyl-3-methoxypropionat
4-(5-Pentylpyrimidinyl-2)-phenyl-2-methyl-3-methoxypropionat
4-(5-Butylpyrimidinyl-2)-phenyl-2-methyl-3-methoxypropionat
4-(5-Nonylpyrimidinyl-2)-phenyl-2-methyl-3-butoxypropionat
4-(5-Octylpyrimidinyl-2)-phenyl-2-methyl-3-butoxypropionat
4-(5-Heptylpyrimidinyl-2)-phenyl-2-methyl-3-butoxypropionat
4-(5-Hexylpyrimidinyl-2)-phenyl-2-methyl-3-butoxypropionat
4-(5-Pentylpyrimidinyl-2)-phenyl-2-methyl-3-butoxypropionat
4-(5-Butylpyrimidinyl-2)-phenyl-2-methyl-3-butoxypropionat
4-(5-Nonylpyrimidinyl-2)-phenyl-2-methyl-3-pentoxypropionat

4-(5-Octylpyrimidinyl-2)-phenyl-2-methyl-3-pentoxypropionat
4-(5-Heptylpyrimidinyl-2)-phenyl-2-methyl-3-pentoxypropionat
4-(5-Hexylpyrimidinyl-2)-phenyl-2-methyl-3-pentoxypropionat
4-(5-Pentylpyrimidinyl-2)-phenyl-2-methyl-3-pentoxypropionat
4-(5-Butylpyrimidinyl-2)-phenyl-2-methyl-3-pentoxypropionat
4-(5-Nonylpyrimidinyl-2)-phenyl-2-methyl-3-heptoxypropionat
4-(5-Octylpyrimidinyl-2)-phenyl-2-methyl-3-heptoxypropionat
4-(5-Heptylpyrimidinyl-2)-phenyl-2-methyl-3-heptoxypropionat
4-(5-Hexylpyrimidinyl-2)-phenyl-2-methyl-3-heptoxypropionat
4-(5-Pentylpyrimidinyl-2)-phenyl-2-methyl-3-heptoxypropionat
4-(5-Butylpyrimidinyl-2)-phenyl-2-methyl-3-heptoxypropionat
4-(5-Nonylpyrazinyl-2)-phenyl-2-methyl-3-propoxypropionat
4-(5-Octylpyrazinyl-2)-phenyl-2-methyl-3-propoxypropionat
4-(5-Heptylpyrazinyl-2)-phenyl-2-methyl-3-propoxypropionat
4-(5-Hexylpyrazinyl-2)-phenyl-2-methyl-3-propoxypropionat
4-(5-Pentylpyrazinyl-2)-phenyl-2-methyl-3-propoxypropionat
4-(5-Butylpyrazinyl-2)-phenyl-2-methyl-3-propoxypropionat
4-(5-Nonylpyridinyl-2)-phenyl-2-methyl-3-propoxypropionat
4-(5-Octylpyridinyl-2)-phenyl-2-methyl-3-propoxypropionat
4-(5-Heptylpyridinyl-2)-phenyl-2-methyl-3-propoxypropionat
4-(5-Hexylpyridinyl-2)-phenyl-2-methyl-3-propoxypropionat
4-(5-Pentylpyridinyl-2)-phenyl-2-methyl-3-propoxypropionat
4-(5-Butylpyridinyl-2)-phenyl-2-methyl-3-propoxypropionat
4-(5-Nonylpyrimidinyl-2)-phenyl-3-methyl-3-propoxypropionat
4-(5-Octylpyrimidinyl-2)-phenyl-3-methyl-3-propoxypropionat
4-(5-Heptylpyrimidinyl-2)-phenyl-3-methyl-3-propoxypropionat
4-(5-Hexylpyrimidinyl-2)-phenyl-3-methyl-3-propoxypropionat
4-(5-Pentylpyrimidinyl-2)-phenyl-3-methyl-3-propoxypropionat
4-(5-Butylpyrimidinyl-2)-phenyl-3-methyl-3-propoxypropionat

Beispiel 6

0.75 g optisch aktives 1-Butyryloxy-2-propanol [erhältlich durch Veresterung von (S)-1,2-Propandiol], 1,7 g p-(p-n-Octylphenyl)-benzoesäurechlorid, 20 ml Toluol und 2,1 ml Pyridin werden 6 Std. am Rückfluß gekocht. Das Pyridinhydrochlorid wird heiß abgesaugt und das Filtrat wie üblich aufgearbeitet. Man erhält optisch aktives 1-Butyryloxy-2-[p-(p-n-octylphenyl)-benzoyloxy]-propan.

Die folgenden optisch aktiven Verbindungen werden analoghergestellt:

1-Butyryloxy-2-[p-(p-nonylphenyl)-benzoyloxy]-propan
1-Butyryloxy-2-[p-(p-heptylphenyl)-benzoyloxy]-propan
1-Butyryloxy-2-[p-(p-hexylphenyl)-benzoyloxy]-propan
1-Butyryloxy-2-[p-(p-pentylphenyl)-benzoyloxy]-propan
1-Butyryloxy-2-[p-(p-butylphenyl)-benzoyloxy]-propan
1-Butyryloxy-2-[p-(p-propylphenyl)-benzoyloxy]-propan
1-Butoxy-2-[p-(p-nonylphenyl)-benzoyloxy]-propan
1-Butoxy-2-[p-(p-octylphenyl)-benzoyloxy]-propan
1-Butoxy-2-[p-(p-heptylphenyl)-benzoyloxy]-propan
1-Butoxy-2-[p-(p-hexylphenyl)-benzoyloxy]-propan
1-Butoxy-2-[p-(p-pentylphenyl)-benzoyloxy]-propan
1-Butoxy-2-[p-(p-butylphenyl)-benzoyloxy]-propan
1-Butoxy-2-[p-(p-propylphenyl)-benzoyloxy]-propan
1-Methoxy-2-[p-(p-nonylphenyl)-benzoyloxy]-propan
1-Methoxy-2-[p-(p-octylphenyl)-benzoyloxy]-propan
1-Methoxy-2-[p-(p-heptylphenyl)-benzoyloxy]-propan
1-Methoxy-2-[p-(p-hexylphenyl)-benzoyloxy]-propan
1-Methoxy-2-[p-(p-pentylphenyl)-benzoyloxy]-propan
1-Methoxy-2-[p-(p-butylphenyl)-benzoyloxy]-propan
1-Methoxy-2-[p-(p-propylphenyl)-benzoyloxy]-propan
1-Heptoxy-2-[p-(p-nonylphenyl)-benzoyloxy]-propan
1-Heptoxy-2-[p-(p-octylphenyl)-benzoyloxy]-propan
1-Heptoxy-2-[p-(p-heptylphenyl)-benzoyloxy]-propan
1-Heptoxy-2-[p-(p-hexylphenyl)-benzoyloxy]-propan
1-Heptoxy-2-[p-(p-pentylphenyl)-benzoyloxy]-propan
1-Heptoxy-2-[p-(p-butylphenyl)-benzoyloxy]-propan
1-Heptoxy-2-[p-(p-propylphenyl)-benzoyloxy]-propan
1-Butoxy-1-[p-(p-nonylphenyl)-benzoyloxy]-ethan
1-Butoxy-1-[p-(p-octylphenyl)-benzoyloxy]-ethan

1-Butoxy-1-[p-(p-heptylphenyl)-benzoyloxy]-ethan
1-Butoxy-1-[p-(p-hexylphenyl)-benzoyloxy]-ethan
1-Butoxy-1-[p-(p-pentylphenyl)-benzoyloxy]-ethan
1-Butoxy-1-[p-(p-butylphenyl)-benzoyloxy]-ethan
1-Butoxy-1-[p-(p-propylphenyl)-benzoyloxy]-ethan
1-Butoxy-1-[p-(5-nonylpyrimidinyl-2)-benzoyloxy]-ethan
1-Butoxy-1-[p-(5-octylpyrimidinyl-2)-benzoyloxy]-ethan
1-Butoxy-1-[p-(5-heptylpyrimidinyl-2)-benzoyloxy]-ethan
1-Butoxy-1-[p-(5-hexylpyrimidinyl-2)-benzoyloxy]-ethan
1-Butoxy-1-[p-(5-pentylpyrimidinyl-2)-benzoyloxy]-ethan
1-Butoxy-1-[p-(5-butylpyrimidinyl-2)-benzoyloxy]-ethan
1-Butoxy-1-[p-(5-propylpyrimidinyl-2)-benzoyloxy]-ethan

Es folgen Beispiele für erfindungsgemäße Flüssigkristall-Phasen mit einem Gehalt an mindestens einer optisch aktiven Verbindung der Formel I. Es ist jeweils der Absolutwert des pc-Produktes angegeben.

Beispiel A

Eine Flüssigkristall-Phase aus 2,0% 1,2-Bis-(p-trans-4-n-heptylcyclohexylbenzoyloxy)-propan, gelöst in RO—TN 404 [handelsübliche nematische Flüssigkristallmischung auf der Basis von Cyanopyrimidinen] zeigt bei Linkshändigkeit der Helixstruktur ein pc-Produkt (p = Ganghöhe im µm; c = Konzentration in Gew.%] von 26,8 µm·Gew.-% bei 12°.

Beispiel B

Die Flüssigkristall-Phase aus Beispiel A zeigt bei Linkshändigkeit der Helixstruktur bei 19° (28°) ein pc-Produkt von 28,0 (33,7) µm·Gew.-%.

Beispiel C

Die Flüssigkristall-Phase aus Beispiel A zeigt bei Linkshändigkeit der Helixstruktur bei 38° (48°) ein pc-Produkt von 40,0 (47,4) µm·Gew.-%.

Beispiel D

Die Flüssigkristall-Phase aus Beispiel A zeigt bei Linkshändigkeit der Helixstruktur bei 58° (79°) ein pc-Produkt von 53,3 (175,6) µm·Gew.-%.

Beispiel E

Die Flüssigkristall-Phase aus Beispiel A zeigt bei Linkshändigkeit der Helixstruktur bei 84° ein pc-Produkt von 377,2 µm·Gew.-%.

Beispiel F

Die Flüssigkristall-Phase aus Beispiel A zeigt bei Rechtshändigkeit der Helixstruktur bei 102° ein pc-Produkt von 187,5 µm·Gew.-%.

Beispiel G

Eine Flüssigkristall-Phase aus 0,2% 1,2-Bis-(p-trans-4-n-heptylcyclohexylbenzoyloxy)-1-phenylethan gelöst in RO—TN 404 zeigt bei Linkshändigkeit der Helixstruktur ein pc-Produkt von 3,7 µm·Gew.-% bei 23°.

Beispiel H

Die Flüssigkristall-Phase aus Beispiel G zeigt bei Linkshändigkeit der Helixstruktur bei 43° (83°) ein pc-Produkt von jeweils 3,7 µm·Gew.-%.

Beispiel I

Eine Flüssigkristall-Phase aus 2,4% 1,2-Bis-(p-trans-4-n-heptylcyclohexylbenzoyloxy)-1-phenylethan gelöst in RO—TN 404 zeigt bei 28° ein pc-Produkt von 4,2 µm·Gew.-%.

Beispiel J

Eine Flüssigkristall-Phase aus 8,0% 1,2-Bis-(p-trans-4-n-heptylcyclohexylbenzoyloxy)-1-phenylethan gelöst in RO—TN 404 zeigt eine Absorptionsbande bei 660 nm.

**Patentansprüche**

1. Optisch aktive Verbindungen der Formel I

$$R^1—X^1—CHR^o—(CH_2)_p—X^2—R^2 \qquad I$$

worin $X^1$ und $X^2$ jeweils unabhängig voneinander —CO—O— oder —O—CO—, eine der Gruppen $X^1$ und $X^2$

auch —O—, wobei $R^1$ und $R^2$ unabhängig voneinander jeweils eine Gruppe $—(A^1—Z)_m—(A^2)_n—Y$ bedeuten, worin

$A^1$ und $A^2$ jeweils unabhängig voneinander eine 1,4-Phenylen-, Pyrimidin-2,5-diyl, Pyrazin-2,5-diyl, Pyridin-2,5-diyl, 1,4-Cyclohexylen-, 1,3-Dioxan-2,5,-diyl-, 1,3-Dithian-2,5-diyl- oder 1,4-Bicyclo(2,2,2)-octylen-Gruppe, wobei diese auch ein- oder mehrfach substituiert sein kann durch F, Cl, Br oder Cn

Z —CO—O—, —O—CO—, $—CH_2CH_2—$, $—OCH_2$, $—CH_2O—$, —CH=N—, —N=CH—, —N=N—, —N(O)=N— oder eine Einfachbindung,

m und n jeweils unabhängig voneinander 0, 1 oder 2 und

Y eine geradkettige oder verzweigte Alkylgruppe mit bis zu 12 C-Atomen, wobei 1 oder 2 nicht benachbarte und nicht endständige $CH_2$-Gruppen durch O-Atome ersetzt sein können, oder falls n 1 oder 2 ist, auch F, Cl, Br oder CN, mit der Maßgabe, daß (m + n) in mindestens einer der Gruppen $R^1$ und $R^2$ 2, 3 oder 4 ist,

p 0 oder 1 ist, und

$R^o$ eine Alkylgruppe mit bis zu 5 C-Atomen, eine Phenylgruppe oder eine Cyclohexylgruppe ist, und der Formeln Ia und If

$$Y—CO—O—CHR^o—CH_2—O—CO—A^1—Y \quad \text{Ia}$$

$$Y—A^1—CO—O—CHR^o—CH_2—O—CO—A^1—Y \quad \text{If}$$

worin Y, $R^o$, und $A^1$ die angegebene Bedeutung haben.

2. 1,2-Bis-(p-ethoxybenzoyloxy)-propan
1,2-Bis-(p-propoxybenzoyloxy)-propan
1,2-Bis-(p-butoxybenzoyloxy)-propan
1,2-Bis-(p-pentoxybenzoyloxy)-propan
1,2-Bis-(p-hexoxybenzoyloxy)-propan
1,2-Bis-(p-heptoxybenzoyloxy)-propan
1,2-Bis-(p-octoxybenzoyloxy)-propan
1,2-Bis-(p-decoxybenzoyloxy)-propan
1,2-Bis-(p-ethylbenzoyloxy)-propan
1,2-Bis-(p-propylbenzoyloxy)-propan
1,2-Bis-(p-butylbenzoyloxy)-propan
1,2-Bis-(p-pentylbenzoyloxy)-propan
1,2-Bis-(p-hexylbenzoyloxy)-propan
1,2-Bis-(p-heptylbenzoyloxy)-propan
1,2-Bis-(p-octylbenzoyloxy)-propan
1,2-Bis-(p-decylbenzoyloxy)-propan
1,2-Bis-(p-1-methylpropylbenzoyloxy)-propan
1,2-Bis-(p-(R)-2-methylpropylbenzoyloxy)-propan
1,2-Bis-(p-(R)-2-methylbutylbenzoyloxy)-propan
1,2-Bis-(p-(S)-3-methylbutylbenzoyloxy)-propan
1,2-Bis-(p-(R)-2-methylpentylbenzoyloxy)-propan
1,2-Bis-(p-2-ethylhexylbenzoyloxy)-propan
1,2-Bis-(p-(S)-2-methylpropoxybenzoyloxy)-propan
1,2-Bis-(p-(R)-2-methylbutoxybenzoyloxy)-propan
1,2-Bis-(p-(S)-3-methylbutoxybenzoyloxy)-propan
1,2-Bis-(p-(R)-2-methylpentoxybenzoyloxy)-propan
1,2-Bis-(p-2-ethylhexoxybenzoyloxy)-propan
1,2-Bis-(p-(R)-2-oxa-3-methylbutylbenzoyloxy)-propan.
1,2-Bis-(p-ethoxybenzoyloxy)-1-phenylethan
1,2-Bis-(p-propoxybenzoyloxy)-1-phenylethan
1,2-Bis-(p-butoxybenzoyloxy)-1-phenylethan
1,2-Bis-(p-pentoxybenzoyloxy)-1-phenylethan
1,2-Bis-(p-hexoxybenzoyloxy)-1-phenylethan
1,2-Bis-(p-heptoxybenzoyloxy)-1-phenylethan
1,2-Bis-(p-octoxybenzoyloxy)-1-phenylethan
1,2-Bis-(p-decoxybenzoyloxy)-1-phenylethan
1,2-Bis-(p-ethylbenzoyloxy)-1-phenylethan
1,2-Bis-(p-propylbenzoyloxy)-1-phenylethan
1,2-Bis-(p-butylbenzoyloxy)-1-phenylethan
1,2-Bis-(p-pentylbenzoyloxy)-1-phenylethan
1,2-Bis-(p-hexylbenzoyloxy)-1-phenylethan
1,2-Bis-(p-heptylbenzoyloxy)-1-phenylethan
1,2-Bis-(p-octylbenzoyloxy)-1-phenylethan
1,2-Bis-(p-decylbenzoyloxy)-1-phenylethan

1,2-Bis-(p-1-methylpropylbenzoyloxy)-1-phenylethan
1,2-Bis-(p-(R)-2-methylpropylbenzoyloxy)-1-phenylethan
1,2-Bis-(p-(R)-2-methylbutylbenzoyloxy)-1-phenylethan
1,2-Bis-(p-(S)-3-methylbutylbenzoyloxy)-1-phenylethan
1,2-Bis-(p-(R)-2-methylpentylbenzoyloxy)-1-phenylethan
1,2-Bis-(p-2-ethylhexylbenzoyloxy)-1-phenylethan
1,2-Bis-(p-(S)-2-methylpropoxybenzoyloxy)-1-phenylethan
1,2-Bis-(p-(R)-2-methylbutoxybenzoyloxy)-1-phenylethan
1,2-Bis-(p-(S)-3-methylbutoxybenzoyloxy)-1-phenylethan
1,2-Bis-(p-(R)-2-methylpentoxybenzoyloxy)-1-phenylethan
1,2-Bis-(p-2-ethylhexoxybenzoyloxy)-1-phenylethan
1,2-Bis-(p-(R)-2-oxa-3-methylbutylbenzoyloxy)-1-phenylethan.
1,2-Bis-(p-ethoxybenzoyloxy)-1-cyclohexylethan
1,2-Bis-(p-propoxybenzoyloxy)-1-cyclohexylethan
1,2-Bis-(p-butoxybenzoyloxy)-1-cyclohexylethan
1,2-Bis-(p-pentoxybenzoyloxy)-1-cyclohexylethan
1,2-Bis-(p-hexoxybenzoyloxy)-1-cyclohexylethan
1,2-Bis-(p-heptoxybenzoyloxy)-1-cyclohexylethan
1,2-Bis-(p-octoxybenzoyloxy)-1-cyclohexylethan
1,2-Bis-(p-decoxybenzoyloxy)-1-cyclohexylethan
1,2-Bis-(p-ethylbenzoyloxy)-1-cyclohexylethan
1,2-Bis-(p-propylbenzoyloxy)-1-cyclohexylethan
1,2-Bis-(p-butylbenzoyloxy)-1-cyclohexylethan
1,2-Bis-(p-pentylbenzoyloxy)-1-cyclohexylethan
1,2-Bis-(p-hexylbenzoyloxy)-1-cyclohexylethan
1,2-Bis-(p-heptylbenzoyloxy)-1-cyclohexylethan
1,2-Bis-(p-octylbenzoyloxy)-1-cyclohexylethan
1,2-Bis-(p-decylbenzoyloxy)-1-cyclohexylethan
1,2-Bis-(p-1-methylpropylbenzoyloxy)-1-cyclohexylethan
1,2-Bis-(p-(R)-2-methylpropylbenzoyloxy)-1-cyclohexylethan
1,2-Bis-(p-(R)-2-methylbutylbenzoyloxy)-1-cyclohexylethan
1,2-Bis-(p-(S)-3-methylbutylbenzoyloxy)-1-cyclohexylethan
1,2-Bis-(p-(R)-2-methylpentylbenzoyloxy)-1-cyclohexylethan
1,2-Bis-(p-2-ethylhexylbenzoyloxy)-1-cyclohexylethan
1,2-Bis-(p-(S)-2-methylpropoxybenzoyloxy)-cyclohexylethan
1,2-Bis-(p-(R)-2-methylbutoxybenzoyloxy)-1-cyclohexylethan
1,2-Bis-(p-(S)-3-methylbutoxybenzoyloxy)-1-cyclohexylethan
1,2-Bis-(p-(R)-2-methylpentoxybenzoyloxy)-1-cyclohexylethan
1,2-Bis-(p-2-ethylhexoxybenzoyloxy)-1-cyclohexylethan
1,2-Bis-(p-(R)-2-oxa-3-methylbutylbenzoyloxy)-1-cyclohexylethan.

3. Optisch aktive Verbindung nach Anspruch 1, dadurch gekennzeichnet, daß

$A^1$ und $A^2$ jeweils unabhängig voneinander eine 1,4-Phenylen-, Pyrimidin-2,5-diyl, Pyrazin-2,5-diyl, Pyridin-2,5-diyl oder 1,4-Cyclohexylen-Gruppe wobei diese auch ein- oder mehrfach substituiert sein kann durch F, Cl, Br oder Cn

Z —CO—O—, —O—CO— oder eine Einfachbindung,

Y eine geradkettige oder verzweigte Alkylgruppe mit biz zu 12 C-Atomen, wobei 1 oder 2 nicht benachbarte und nicht endständige $CH_2$-Gruppen durch O-Atome ersetzt sein können, oder falls n 1 oder 2 ist, auch CN, und

$R^o$ eine Methylgruppe, eine Phenylgruppe oder eine Cyclohexylgruppe ist.

4. Optisch aktive Verbindungen nach Anspruch 3, dadurch gekennzeichnet, daß

$A^1$ und $A^2$ jeweils unabhängig voneinander eine 1,4-Phenylen-, Pyrimidin-2,5-diyl, Pyrazin-2,5-diyl, Pyridin-2,5-diyl oder 1,4-Cyclohexylen-Gruppe bedeuten.

5. Optisch aktive Verbindung nach Anspruch 1, 3 oder 4 der Formel I'

$$Y—X^1—CHR^o—(CH_2)_p—X^2—(A^1—Z)_m—(A^2)_n—Y \qquad I'$$

worin Y, $X^1$, $X^2$, $R^o$, p, $A^1$, $A^2$, Z, m und n die oben angegebene Bedeutung haben.

6. Optisch aktive Verbindung nach Anspruch 1, 3 oder 4 der Formel I''

$$Y—(A^2)_n—(Z—A^1)_m—X^1—CHR^o—(CH_2)_p—X^2—Y \qquad I''$$

worin Y, $X^1$, $X^2$, $R^o$, p, $A^1$, $A^2$, Z, m und n die oben angegebene Bedeutung haben.

7. Optisch aktive Verbindung nach Anspruch 1, 3 oder 4 der Formel Ib′

$$Y—X^1—CHR^o—(CH_2)_p—X^2—A^1—A^2—Y \qquad Ib'$$

worin Y, $X^1$, $X^2$, $R^o$, p, $A^1$ und $A^2$ die oben angegebene Bedeutung haben.

8. Optisch aktive Verbindung nach Anspruch 1, 3 oder 4 der Formel Ib″

$$Y—A^2—A^1—X^1—CHR^o—(CH_2)_p—X^2—Y \qquad Ib''$$

worin Y, $X^1$, $X^2$, $R^o$, p, $A^1$ und $A^2$ die oben angegebene Bedeutung haben.

9. Optisch aktive Verbindung nach Anspruch 1, 3 oder 4 der Formel Ic′

$$Y—X^1—CHR^o—(CH_2)_p—X^2—A^1—Z—A^2—Y \qquad Ic'$$

worin Y, $X^1$, $X^2$, $R^o$, p, $A^1$, $A^2$ und Z die oben angegebene Bedeutung haben.

10. Optisch aktive Verbindung nach Anspruch 1, 3 oder 4 der Formel Ic″

$$Y—A^2—Z—A^1—X^1—CHR^o—(CH_2)_p—X^2—Y \qquad Ic''$$

worin Y, $X^1$, $X^2$, $R^o$, p, $A^1$, $A^2$ und Z die oben angegebene Bedeutung haben.

11. Optisch aktive Verbindung nach Anspruch 1, 3, 4, 5 oder 6, worin $A^1$ und $A^2$ 1,4-Phenylen bedeutet.

12. Optisch aktive Verbindung nach Anspruch 1, 3, 4, 5 oder 6, worin eine der Gruppen $A^1$ und $A^2$ 1,4-Phenylen bedeutet und die andere Pyrimidin-2,5-diyl, Pyrazin-2,5-diyl oder Pyridin-2,5-diyl ist.

13. Optisch aktive Verbindung nach Anspruch 7 oder 8, worin $—A^1—A^2$

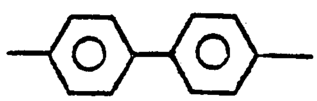 , 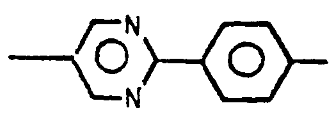 ,

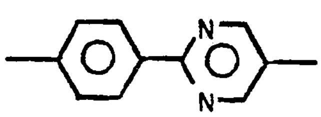 , 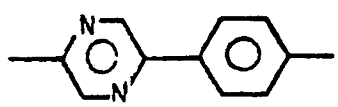 ,

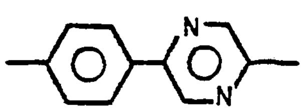 , 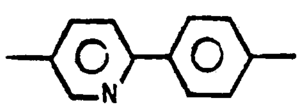 oder 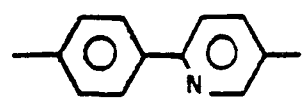

bedeutet.

14. Optisch aktive Verbindung nach einem der Ansprüche 1 oder 3, bis 13, worin $R^o$ Methyl ist.

15. Optisch aktive Verbindung nach einem der Ansprüche 1 oder 3, bis 14, worin Y eine geradkettige Alkylgruppe mit bis zu 7 C-Atomen ist.

16. Optisch aktive Verbindung nach einem der Ansprüche 1 oder 3, bis 15, worin p 1 bedeutet.

17. Optisch aktive Verbindung nach einem der Ansprüche 1 oder 3, bis 16, worin $X^1$ —CO—O— bedeutet.

18. Optisch aktive Verbindung nach einem der Ansprüche 1 oder 3, bis 17, worin $X^2$ —O—CO— bedeutet.

19. Flüssigkristall-Phase, gekennzeichnet durch einen Gehalt an mindestens einer optisch aktiven Verbindung der Formel I

$$R^1—X^1—CHR^o—(CH_2)_p—X^2—R^2 \qquad I$$

worin $X^1$ und $X^2$ jeweils unabhängig voneinander —CO—O— oder —O—CO—, eine der Gruppen $X^1$ und $X^2$ auch —O—, wobei $R^1$ und $R^2$ unabhängig voneinander jeweils eine Gruppe $—(A^1—Z)_m—(A^2)_n—Y$ bedeuten, worin

$A^1$ und $A^2$ jeweils unabhängig voneinander eine 1,4-Phenylen-, Pyrimidin-2,5-diyl, Pyrazin-2,5-diyl, Pyridin-2,5-diyl, 1,4-Cyclohexylen-, 1,3-Dioxan-2,5-diyl-, 1,3-Dithian-2,5-diyl- oder 1,4-Bicyclo(2,2,2)-octylen-Gruppe, wobei diese auch ein- oder mehrfach substituiert sein kann durch F, Cl, Br, Cn und/oder Alkylgruppen mit bis zu 12 C-Atomen, wobei in den Alkylgruppen 1 oder 2 nicht benachbarte $CH_2$-Gruppen durch O-Atome ersetzt sein können,

Z —CO—O—, —O—CO—, $—CH_2CH_2—$, $—OCH_2$, $—CH_2O—$, —CH=N—, —N=CH—, —N=N—, —N(O)=N— oder eine Einfachbindung,

m und n jeweils unabhängig voneinander 0, 1 oder 2 und

Y eine geradkettige oder verzweigte Alkylgruppe mit bis zu 12 C-Atomen, wobei 1 oder 2 nicht benachbarte und nicht endständige $CH_2$-Gruppen durch O-Atome ersetzt sein können, oder falls n 1 oder 2 ist, auch F, Cl, Br oder CN,

p 0 oder 1 ist, und

$R^o$ eine Alkylgruppe mit bis zu 5 C-Atomen, eine Phenylgruppe oder eine Cyclohexylgruppe ist.

20. Flüssigkristall-Phase nach Anspruch 19, gekennzeichnet durch einen Gehalt an mindestens einer optisch aktiven Verbindung nach einem der Ansprüche 3 bis 18.

21. Flüssigkristallanzeigeelemente, enthaltend eine Flüssigkristall-Phase nach Anspruch 19.

22. Verwendung der Verbindungen der Formel I gemäß Anspruch 1 zur Temperaturkompensation in Flüssigkristall-Phasen.

23. Verfahren zur Temperaturkompensation in Flüssigkristallanzeigeelementen, dadurch gekennzeichnet, daß man der Flüssigkristall-Phase mindestens 0,05% an mindestens einer Verbindung der Formel I zumischt.

**Revendications**

1. Composés optiquement actifs de formule I

$$R^1—X^1—CHR^o—(CH_2)_p—X^2—R^2 \qquad I$$

où $X^1$ et $X^2$ représentent chacun indépendamment l'un de l'autre —CO—O— ou —O—CO—, l'un des groupes $X^1$ et $X^2$ représente également —O—, où $R^1$ et $R^2$ représentent chacun indépendamment l'un de l'autre un groupe $—(A^1—Z)_m—(A^2)_n—Y$, où

$A^1$ et $A^2$ représentent chacun indépendamment l'un de l'autre un groupe 1,4-phénylène, pyrimidine-2,5-diyle, pyrazine-2,5-diyle, pyridine-2,5-diyle, 1,4-cyclohexylène, 1,3-dioxanne-2,5-diyle, 1,3-dithian-2,5-diyle ou 1,4-bicyclo(2,2,2)-octylène, où ceux-ci peuvent également être mono- ou polysubstitués par F, Cl, Br, CN et

Z représente —CO—O—, —O—CO—, $—CH_2CH_2—$, $—OCH_2$, $—CH_2O—$, —CH=N—, —N=CH—, —N=N—, —N(O)=N— ou une liaison simple,

m et n représentent chacun indépendamment l'un de l'autre 0, 1 ou 2 et

Y représente un groupe alcoyle à chaîne droite ou ramifiée ayant jusqu'à 12 atomes de carbone où un ou deux groupes $CH_2$ non voisins et ne se situant pas en position finale peuvent être remplacés par des atomes de O, ou si n vaut 1 ou 2, également F, Cl, Br ou CN,

avec cette précision que (m + n) dans au moins un des groupes $R^1$ et $R^2$ vaut 2, 3 ou 4,

p vaut 0 ou 1 et

$R^o$ représente un groupe alcoyle ayant jusqu'à 5 atomes de carbone, un groupe phényle ou un groupe cyclohexyle,

et de formules Ia et If

$$Y—CO—O—CHR^o—CH_2—O—CO—A^1—Y \qquad Ia$$

$$Y—A^1—CO—O—CHR^o—CH_2—O—CO—A^1—Y \qquad If$$

où Y, $R^o$ et $A^1$ ont la signification donnée ci-dessus.

2. 1,2-bis-(p-éthoxybenzoyloxy)-propane
1,2-bis-(p-propoxybenzoyloxy)-propane
1,2-bis-(p-butoxybenzoyloxy)-propane
1,2-bis-(p-pentoxybenzoyloxy)-propane
1,2-bis-(p-hexoxybenzoyloxy)-propane
1,2-bis-(p-heptoxybenzoyloxy)-propane
1,2-bis-(p-octoxybenzoyloxy)-propane
1,2-bis-(p-décoxybenzoyloxy)-propane
1,2-bis-(p-éthylbenzoyloxy)-propane
1,2-bis-(p-propylbenzoyloxy)-propane
1,2-bis-(p-butylbenzoyloxy)-propane
1,2-bis-(p-pentylbenzoyloxy)-propane
1,2-bis-(p-hexylbenzoyloxy)-propane
1,2-bis-(p-heptylbenzoyloxy)-propane
1,2-bis-(p-octylbenzoyloxy)-propane
1,2-bis-(p-décylbenzoyloxy)-propane
1,2-bis-(p-1-méthylpropylbenzoyloxy)-propane

1,2-bis-(p-(R)-2-méthylpropylbenzoyloxy)-propane
1,2-bis-(p-(R)-2-méthylbutylbenzoyloxy)-propane
1,2-bis-(p-(S)-3-méthylbutylbenzoyloxy)-propane
1,2-bis-(p-(R)-2-méthylpentylbenzoyloxy)-propane
1,2-bis-(p-2-éthylhexylbenzoyloxy)-propane
1,2-bis-(p-(S)-2-méthylpropoxybenzoyloxy)-propane
1,2-bis-(p-(R)-2-méthylbutoxybenzoyloxy)-propane
1,2-bis-(p-(S)-3-méthylbutoxybenzoyloxy)-propane
1,2-bis-(p-(R)-2-méthylpentoxybenzoyloxy)-propane
1,2-bis-(p-2-éthylhexoxybenzoyloxy)-propane
1,2-bis-(p-(R)-2-oxa-3-méthylbutylbenzoyloxy)-propane.
1,2-bis-(p-éthoxybenzoyloxy)-1-phényléthane
1,2-bis-(p-propoxybenzoyloxy)-1-phényléthane
1,2-bis-(p-butoxybenzoyloxy)-1-phényléthane
1,2-bis-(p-pentoxybenzoyloxy)-1-phéyléthane
1,2-bis-(p-hexoxybenzoyloxy)-1-phényléthane
1,2-bis-(p-heptoxybenzoyloxy)-1-phényléthane
1,2-bis-(p-octoxybenzoyloxy)-1-phényléthane
1,2-bis-(p-décoxybenzoyloxy)-1-phényléthane
1,2-bis-(p-éthylbenzoyloxy)-1-phényléthane
1,2-bis-(p-propylbenzoyloxy)-1-phényléthane
1,2-bis-(p-butylbenzoyloxy)-1-phényléthane
1,2-bis-(p-pentylbenzoyloxy)-1-phényléthane
1,2-bis-(p-hexylbenzoyloxy)-1-phényléthane
1,2-bis-(p-heptylbenzoyloxy)-1-phényléthane
1,2-bis-(p-octylbenzoyloxy)-1-phényléthane
1,2-bis-(p-décylbenzoyloxy)-1-phényléthane
1,2-bis-(p-1-méthylpropylbenzoyloxy)-1-phényléthane
1,2-bis-(p-(R)-2-méthylpropylbenzoyloxy)-1-phényléthane
1,2-bis-(p-(R)-2-méthylbutylbenzoyloxy)-1-phényléthane
1,2-bis-(p-(S)-3-méthylbutylbenzoyloxy)-1-phényléthane
1,2-bis-(p-(R)-2-méthylpentylbenzoyloxy)-1-phényléthane
1,2-bis-(p-2-éthylhexylbenzoyloxy)-1-phényléthane
1,2-bis-(p-(S)-2-méthylpropoxybenzoyloxy)-1-phényléthane
1,2-bis-(p-(R)-2-méthylbutoxybenzoyloxy)-1-phényléthane
1,2-bis-(p-(S)-3-méthylbutoxybenzoyloxy)-1-phényléthane
1,2-bis-(p-(R)-2-méthylpentoxybenzoyloxy)-1-phényléthane
1,2-bis-(p-2-éthylhexoxybenzoyloxy)-1-phényléthane
1,2-bis-(p-(R)-2-oxa-3-méthylbutylbenzoyloxy)-1-phényléthane.
1,2-bis-(p-éthoxybenzoyloxy)-1-cyclohexyléthane
1,2-bis-(p-propoxybenzoyloxy)-1-cyclohexyléthane
1,2-bis-(p-butoxybenzoyloxy)-1-cyclohexyléthane
1,2-bis-(p-pentoxybenzoyloxy)-1-cyclohexyléthane
1,2-bis-(p-hexoxybenzoyloxy)-1-cyclohexyléthane
1,2-bis-(p-heptoxybenzoyloxy)-1-cyclohexyléthane
1,2-bis-(p-octoxybenzoyloxy)-1-cyclohexyléthane
1,2-bis-(p-décoxybenzoyloxy)-1-cyclohexyléthane
1,2-bis-(p-éthylbenzoyloxy)-1-cyclohexyléthane
1,2-bis-(p-propylbenzoyloxy)-1-cyclohexyléthane
1,2-bis-(p-butylbenzoyloxy)-1-cyclohexyléthane
1,2-bis-(p-pentylbenzoyloxy)-1-cyclohexyléthane
1,2-bis-(p-hexylbenzoyloxy)-1-cyclohexyléthane
1,2-bis-(p-heptylbenzoyloxy)-1-cyclohexyléthane
1,2-bis-(p-octylbenzoyloxy)-1-cyclohexyléthane
1,2-bis-(p-déylbenzoyloxy)-1-cyclohexyléthane
1,2-bis-(p-1-méthylpropylbenzoyloxy)-1-cyclohexyléthane
1,2-bis-(p-(R)-2-méthylpropylbenzoyloxy)-1-cyclohexyléthane
1,2-bis-(p-(R)-2-méthylbutylbenzoyloxy)-1-cyclohexyléthane
1,2-bis-(p-(S)-3-méthylbutylbenzoyloxy)-1-cyclohexyléthane
1,2-bis-(p-(R)-2-méthylpentylbenzoyloxy)-1-cyclohexyléthane
1,2-bis-(p-2-éthylhexylbenzoyloxy)-1-cyclohexyléthane
1,2-bis-(p-(S)-2-méthylpropoxybenzoyloxy)-cyclohexyléthane
1,2-bis-(p-(R)-2-méthylbutoxybenzoyloxy)-1-cyclohexyléthane
1,2-bis-(p-(S)-3-méthylbutoxybenzoyloxy)-1-cyclohexyléthane
1,2-bis-(p-(R)-2-méthylpentoxybenzoyloxy)-1-cyclohexyléthane

1,2-bis-(p-2-éthylhexoxybenzoyloxy)-1-cyclohexyléthane

1,2-bis-(p-(R)-2-oxa-3-méthylbutylbenzoyloxy)-1-cyclohexyléthane.

3. Composés optiquement actifs selon la revendication 1, caractérisés en ce que

$A^1$ et $A^2$ représentent chacun indépendamment l'un de l'autre un groupe 1,4-phénylène, pyrimidine-2,5-diyle, pyrazine-2,5-diyle, pyridine-2,5-diyle, ou 1,4-cyclohexylène, où ceux-ci peuvent également être mono- ou polysubstitutés par F, Cl, Br, ou CN

Z représente —CO—O—, —O—CO—, ou une liaison simple,

Y représente un groupe alcoyle à chaîne droite ou ramifiée ayant jusqu'à 12 atomes de carbone, où un ou deux groupes $CH_2$ non voisins et non en position finale peuvent être remplacés par des atomes de 0, ou bien, si n vaut 1 ou 2, également CN et

R° représente un groupe méthyle, un groupe phényle ou un groupe cyclohexyle.

4. Composés optiquement actifs selon la revendication 3, caractérisés en ce que:

$A^1$ et $A^2$ représentent chacun indépendamment l'un de l'autre un groupe 1,4-phénylène, pyrimidine-2,5-diyle, pyrazine-2,5-diyle, pyridine-2,5-diyle ou 1,4-cyclohexylène.

5. Composés optiquement actifs selon la revendication 1, 3 ou 4, de formule I'

$$Y—X^1—CHR^\circ—(CH_2)_p—X^2—(A^1—Z)_m—(A^2)_n—Y \qquad I'$$

où Y, $X^1$, $X^2$, R°, p, $A^1$, $A^2$, Z, m et n ont la signification donnée ci-dessus.

6. Composé optiquement actif selon la revendication 1, 3 ou 4 de formule I''

$$Y—(A^2)_n—(Z—A^1)_m—X^1—CHR^\circ—(CH_2)_p—X^2—Y \qquad I''$$

où Y, $X^1$, $X^2$, R°, p, $A^1$, $A^2$, Z, m et n ont la signification donnée ci-dessus.

7. Composé optiquement actif selon la revendication 1, 3 ou 4 de formule Ib'

$$Y—X^1—CHR^\circ—(CH_2)_p—X^2—A^1—A^2—Y \qquad Ib'$$

où Y, $X^1$, $X^2$, R°, p, $A^1$ et $A^2$ ont la signification donnée ci-dessus.

8. Composé optiquement actif selon la revendication 1, 3 ou 4, de formule Ib''

$$Y—A^2—A^1—X^1—CHR^\circ—(CH_2)_p—X^2—Y \qquad Ib''$$

où Y, $X^1$, $X^2$, R°, p, $A^1$ et $A^2$ ont la signification donnée ci-dessus.

9. Composé optiquement actif selon la revendication 1, 3, ou 4, de formule Ic'

$$Y—X^1—CHR^\circ—(CH_2)_p—X^2—A^1—Z—A^2—Y \qquad Ic'$$

où Y, $X^1$, $X^2$, R°, p, $A^1$, $A^2$ et Z ont la signification donnée ci-dessus.

10. Composé optiquement actif selon la revendication 1, 3 ou 4, de formule Ic''

$$Y—A^2—Z—A^1—X^1—CHR^\circ—(CH_2)_p—X^2—Y \qquad Ic''$$

où Y, $X^1$, $X^2$, R°, p, $A^1$, $A^2$ et Z ont la signification donnée ci-dessus.

11. Composé optiquement actif selon la revendication 1, 3, 4, 5 ou 6 où $A^1$ et $A^2$ représentent un groupe 1,4-phénylène.

12. Composé optiquement actif selon la revendication 1, 3, 4, 5 ou 6 où l'une groupes $A^1$ et $A^2$ représente un 1,4-phénylène et l'autre est un pyrimidine-2,5-diyle, pyrazine-2,5-diyle ou pyridine-2,5-diyle.

13. Composé optiquement actif selon la revendication 7 ou 8, où $—A^1—A^2$ représente

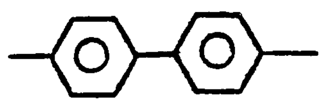 , 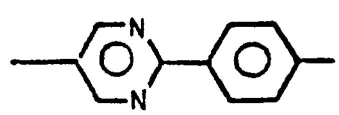 ,

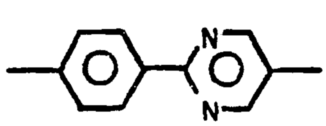 , 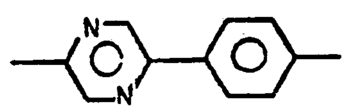 ,

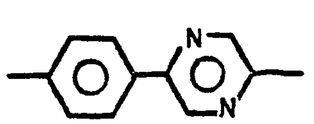 , 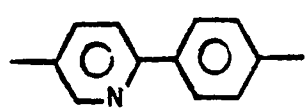 ou 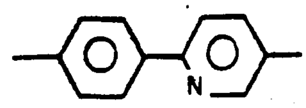

14. Composé optiquement actif selon l'une des revendications 1 ou 3 à 13 où R° est un méthyle.

15. Composé optiquement actif selon l'une des revendications 1 ou 3 à 14, où Y est un groupe alcoyle à chaîne droite ayant jusqu'à 7 atomes de carbone.

16. Composé optiquement actif selon l'une des revendications 1 ou 3 à 15, où p vaut 1.

17. Composé optiquement actif selon l'une des revendications 1 ou 3 à 16 ou $X^1$ représente —CO—O—.

18. Composé optiquement actif selon l'une des revendications 1 ou 3 à 17, où $X^2$ représente —O—CO—.

19. Phase à cristaux liquides caractérisée en ce qu'elle contient au moins un composé optiquement actif de formule I

$$R^1—X^1—CHR^o—(CH_2)_p—X^2—R^2 \qquad I$$

où $X^1$ et $X^2$ représentent chacun indépendamment l'un de l'autre —CO—O— ou —O—CO—, l'un des groupes $X^1$ et $X^2$ représente également —O—, où $R^1$ et $R^2$ représentent chacun indépendamment l'un de l'autre un groupe $—(A^1—Z)_m—(A^2)_n—Y$, où

$A^1$ et $A^2$ représentent chacun indépendamment l'un de l'autre un groupe 1,4-phénylène, pyrimidine-2,5-diyle, pyrazine-2,5-diyle, pyridine-2,5-diyle, 1,4-cyclohexylène, 1,3-dioxanne-2,5-diyle, 1,3-dithian-2,5-diyle ou 1,4-bicyclo(2,2,2)-octylène, où celui-ci peut également être mono- ou polysubstitué par F, Cl, Br, CN et/ou des groupes alcoyle ayant jusqu'à 12 atomes de carbone, où dans les groupes alcoyle un ou deux groupes $CH_2$ non voisins peuvent être remplacés par des atomes de O,

Z représente —CO—O—, —O—CO—, $—CH_2CH_2—$, $—OCH_2$, $—CH_2O—$, —CH=N—, —N=CH—, —N=N—, —N(O)=N— ou une liaison simple,

m et n représentent chacun indépendamment l'une de l'autre 0, 1 ou 2 et

Y représentent un groupe alcoyle à chaîne droite ou ramifiée ayant jusqu'à 12 atomes de carbone, où un ou deux groupes $CH_2$ non voisins et non en position finale peuvent être remplacés par des atomes de O, ou bien si n vaut 1 ou 2, également F, Cl, Br ou CN,

p vaut 0 ou 1 et

R° représente un groupe alcoyle ayant jusqu'à 5 atomes de carbone, un groupe phényle ou un groupe cyclohexyle.

20. Phase à cristaux liquides selon la revendication 19, caractérisé en ce qu'elle contient au moins un composé optiquement actif selon l'une des revendications 3 à 18.

21. Eléments d'affichage à cristaux liquides contenant une phase à cristaux liquides selon la revendication 19.

22. Application du composé de formule I selon la revendication 1 à la compensation de température dans les phases à cristaux liquides.

23. Procédé de compensation de température dans les éléments d'affichage à cristaux liquides caractérisé en ce qu'on mélange à la phase à cristaux liquides au moins 0,05% d'au moins un composé de formule I.

## Claims

1. Optically active compounds of the formula I

$$R^1—X^1—CHR^o—(CH_2)_p—X^2—R^2 \qquad I$$

in which $X^1$ and $X^2$, independently of one another, are each —CO—O— or —O—CO—, one of the groups $X^1$ and $X^2$ is also —O—, in which $R^1$ and $R^2$, independently of one another, are each a group $—(A^1—Z)_m—(A^2)_n—Y$, wherein

$A^1$ and $A^2$, independently of one another, [lacuna] each a 1,4-phenylene, pyrimidine-2,5-diyl, pyrazine-2,5-diyl, pyridine-2,5-diyl, 1,4-cyclohexylene, 1,3-dioxane-2,5-diyl, 1,3-dithiane-2,5-diyl or 1,4-bicyclo[2.2.2.]octylene group, which can also be monosubstituted or polysubstituted by F, Cl, Br or CN

Z is —CO—O—, —O—CO—, $—CH_2CH_2—$, $—OCH_2$, $—CH_2O—$, —CH=N—, —N=CH—, —N=N—, —N(O)=N— or a single bond,

m and n, independently of one another, [lacuna] each 0, 1 or 2, and

Y is a straight-chain or branched alkyl group of up to 12 C atoms, in which 1 or 2 non-adjacent and non-terminal $CH_2$ groups can be replaced by O atoms, or, if n is 1 or 2, is also F, Cl, Br or CN, with the proviso that (m + n) in at least one of the groups $R^1$ and $R^2$ is 2, 3 or 4,

p is 0 or 1 and

R° is an alkyl group of up to 5 C atoms, a phenyl group or a cyclohexyl group,

and of the formulae Ia and If

$$Y—CO—O—CHR^o—CH_2—O—CO—A^1—Y \qquad Ia$$

$$Y—A^1—CO—O—CHR^o—CH_2—O—CO—A^1—Y \qquad If$$

in which Y, R°, and $A^1$ have the meaning given.

2. 1,2-Bis-(p-ethoxybenzoyloxy)-propane
1,2-bis-(p-propoxybenzoyloxy)-propane
1,2-bis-(p-butoxybenzoyloxy)-propane
1,2-bis-(p-pentoxybenzoyloxy)-propane
1,2-bis-(p-hexoxybenzoyloxy)-propane
1,2-bis-(p-heptoxybenzoyloxy)-propane
1,2-bis-(p-octoxybenzoyloxy)-propane
1,2-bis-(p-decoxybenzoyloxy)-propane
1,2-bis-(p-ethylbenzoyloxy)-propane
1,2-bis-(p-propylbenzoyloxy)-propane
1,2-bis-(p-butylbenzoyloxy)-propane
1,2-bis-(p-pentylbenzoyloxy)-propane
1,2-bis-(p-hexylbenzoyloxy)-propane
1,2-bis-(p-heptylbenzoyloxy)-propane
1,2-bis-(p-octylbenzoyloxy)-propane
1,2-bis-(p-decylbenzoyloxy)-propane
1,2-bis-(p-1-methylpropylbenzoyloxy)-propane
1,2-bis-(p-(R)-2-methylpropylbenzoyloxy)-propane
1,2-bis-(p-(R)-2-methylbutylbenzoyloxy)-propane
1,2-bis-(p-(S)-3-methylbutylbenzoyloxy)-propane
1,2-bis-(p-(R)-2-methylpentylbenzoyloxy)-propane
1,2-bis-(p-2-ethylhexylbenzoyloxy)-propane
1,2-bis-(p-(S)-2-methylpropoxybenzoyloxy)-propane
1,2-bis-(p-(R)-2-methylbutoxybenzoyloxy)-propane
1,2-bis-(p-(S)-3-methylbutoxybenzoyloxy)-propane
1,2-bis-(p-(R)-2-methylpentoxybenzoyloxy)-propane
1,2-bis-(p-2-ethylhexoxybenzoyloxy)-propane
1,2-bis-(p-(R)-2-oxa-3-methylbutylbenzoyloxy)-propane
1,2-bis-(p-ethoxybenzoyloxy)-1-phenylethane
1,2-bis-(p-propoxybenzoyloxy)-1-phenylethane
1,2-bis-(p-butoxybenzoyloxy)-1-phenylethane
1,2-bis-(p-pentoxybenzoyloxy)-1-phenylethane
1,2-bis-(p-hexoxybenzoyloxy)-1-phenylethane
1,2-bis-(p-heptoxybenzoyloxy)-1-phenylethane
1,2-bis-(p-octoxybenzoyloxy)-1-phenylethane
1,2-bis-(p-decoxybenzoyloxy)-1-phenylethane
1,2-bis-(p-ethylbenzoyloxy)-1-phenylethane
1,2-bis-(p-propylbenzoyloxy)-1-phenylethane
1,2-bis-(p-butylbenzoyloxy)-1-phenylethane
1,2-bis-(p-pentylbenzoyloxy)-1-phenylethane
1,2-bis-(p-hexylbenzoyloxy)-1-phenylethane
1,2-bis-(p-heptylbenzoyloxy)-1-phenylethane
1,2-bis-(p-octylbenzoyloxy)-1-phenylethane
1,2-bis-(p-decylbenzoyloxy)-1-phenylethane
1,2-bis-(p-1-methylpropylbenzoyloxy)-1-phenylethane
1,2-bis-(p-(R)-2-methylpropylbenzoyloxy)-1-phenylethane
1,2-bis-(p-(R)-2-methylbutylbenzoyloxy)-1-phenylethane
1,2-bis-(p-(S)-3-methylbutylbenzoyloxy)-1-phenylethane
1,2-bis-(p-(R)-2-methylpentylbenzoyloxy)-1-phenylethane
1,2-bis-(p-2-ethylhexylbenzoyloxy)-1-phenylethane
1,2-bis-(p-(S)-2-methylpropoxybenzoyloxy)-1-phenylethane
1,2-bis-(p-(R)-2-methylbutoxybenzoyloxy)-1-phenylethane
1,2-bis-(p-(S)-3-methylbutoxybenzoyloxy)-1-phenylethane
1,2-bis-(p-(R)-2-methylpentoxybenzoyloxy)-1-phenylethane
1,2-bis-(p-2-ethylhexoxybenzoyloxy)-1-phenylethane
1,2-bis-(p-(R)-2-oxa-3-methylbutylbenzoyloxy)-1-phenylethane
1,2-bis-(p-ethoxybenzoyloxy)-1-cyclohexylethane
1,2-bis-(p-propoxybenzoyloxy)-1-cyclohexylethane
1,2-bis-(p-butoxybenzoyloxy)-1-cyclohexylethane
1,2-bis-(p-pentoxybenzoyloxy)-1-cyclohexylethane
1,2-bis-(p-hexoxybenzoyloxy)-1-cyclohexylethane
1,2-bis-(p-heptoxybenzoyloxy)-1-cyclohexylethane
1,2-bis-(p-octoxybenzoyloxy)-1-cyclohexylethane
1,2-bis-(p-decoxybenzoyloxy)-1-cyclohexylethane
1,2-bis-(p-ethylbenzoyloxy)-1-cyclohexylethane

1,2-bis-(p-propylbenzoyloxy)-1-cyclohexylethane
1,2-bis-(p-butylbenzoyloxy)-1-cyclohexylethane
1,2-bis-(p-pentylbenzoyloxy)-1-cyclohexylethane
1,2-bis-(p-hexylbenzoyloxy)-1-cyclohexylethane
1,2-bis-(p-heptylbenzoyloxy)-1-cyclohexylethane
1,2-bis-(p-octylbenzoyloxy)-1-cyclohexylethane
1,2-bis-(p-decylbenzoyloxy)-1-cyclohexylethane
1,2-bis-(p-1-methylpropylbenzoyloxy)-1-cyclohexylethane
1,2-bis-(p-(R)-2-methylpropylbenzoyloxy)-1-cyclohexylethane
1,2-bis-(p-(R)-2-methylbutylbenzoyloxy)-1-cyclohexylethane
1,2-bis-(p-(S)-3-methylbutylbenzoyloxy)-1-cyclohexylethane
1,2-bis-(p-(R)-2-methylpentylbenzoyloxy)-1-cyclohexylethane
1,2-bis-(p-2-ethylhexylbenzoyloxy)-1-cyclohexylethane
1,2-bis-(p-(S)-2-methylpropoxybenzoyloxy)-cyclohexylethane
1,2-bis-(p-(R)-2-methylbutoxybenzoyloxy)-1-cyclohexylethane
1,2-bis-(p-(S)-3-methylbutoxybenzoyloxy)-1-cyclohexylethane
1,2-bis-(p-(R)-2-methylpentoxybenzoyloxy)-1-cyclohexylethane
1,2-bis-(p-2-ethylhexoxybenzoyloxy)-1-cyclohexylethane
1,2-bis-(p-(R)-2-oxa-3-methylbutylbenzoyloxy)-1-cyclohexylethane.

3. Optically active compounds according to Claim 1, characterized in that

$A^1$ and $A^2$, independently of one another, [lacuna] each a 1,4-phenylene, pyrimidine-2,5-diyl, pyrazine-2,5-diyl, pyridine-2,5-diyl or 1,4-cyclohexylene group, which can also be monosubstituted or polysubstituted by F, Cl, Br or CN,

Z is a —CO—O—, —O—CO—, or a single bond,

Y is a straight-chain or branched alkyl group of up to 12 C atoms, in which 1 or 2 non-adjacent and non-terminal $CH_2$ groups can be replaced by O atoms, or, if n is 1 or 2, is also CN and

$R^\circ$ is a methyl group, a phenyl group or a cyclohexyl group.

4. Optically active compounds according to Claim 3, characterized in that

$A^1$ and $A^2$, independently of one another, are each a 1,4-phenylene, pyrimidine-2,5-diyl, pyrazine-2,5-diyl, pyridine-2,5-diyl or 1,4-cyclohexylene group.

5. Optically active compound according to Claim 1, 3 or 4 of the formula I'

$$Y—X^1—CHR^\circ—(CH_2)_p—X^2—(A^1—Z)_m—(A^2)_n—Y \qquad I'$$

in which Y, $X^1$, $X^2$, $R^\circ$, p, $A^1$, $A^2$, Z, m and n have the abovementioned meaning.

6. Optically active compound according to Claim 1, 3 or 4 of the formula I''

$$Y—(A^2)_n—(Z—A^1)_m—X^1—CHR^\circ—(CH_2)_p—X^2—Y \qquad I''$$

in which Y, $X^1$, $X^2$, $R^\circ$, p, $A^1$, $A^2$, Z, m and n have the abovementioned meaning.

7. Optically active compound according to Claim 1, 3 or 4 of the formula Ib'

$$Y—X^1—CHR^\circ—(CH_2)_p—X^2—A^1—A^2—Y \qquad Ib'$$

in which Y, $X^1$, $X^2$, $R^\circ$, p, $A^1$ and $A^2$ have the abovementioned meaning.

8. Optically active compound according to Claim 1, 3 or 4 of the formula Ib''

$$Y—A^2—A^1—X^1—CHR^\circ—(CH_2)_p—X^2—Y \qquad Ib''$$

in which Y, $X^1$, $X^2$, $R^\circ$, p, $A^1$ and $A^2$ have the abovementioned meaning.

9. Optically active compound according to Claim 1, 3 or 4 of the formula Ic'

$$Y—X^1—CHR^\circ—(CH_2)_p—X^2—A^1—Z—A^2—Y \qquad Ic'$$

in which Y, $X^1$, $X^2$, $R^\circ$, p, $A^1$, $A^2$ and Z have the abovementioned meaning.

10. Optically active compound according to Claim 1, 3 or 4 of the formula Ic''

$$Y—A^2—Z—A^1—X^1—CHR^\circ—(CH_2)_p—X^2—Y \qquad Ic''$$

in which Y, $X^1$, $X^2$, $R^\circ$, p, $A^1$, $A^2$ and Z have the abovementioned meaning.

11. Optically active compound according to Claim 1, 3, 4, 5 or 6, in which $A^1$ and $A^2$ is [sic] 1,4-phenylene.

12. Optically active compound according to Claim 1, 3, 4, 5 or 6, in which one of the groups $A^1$ and $A^2$ is 1,4-phenylene and the other is pyrimidine-2,5-diyl, pyrazine-2,5-diyl or pyridine-2,5-diyl.

13. Optically active compound according to Claim 7 or 8, in which $—A^1—A^2$ is

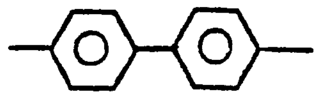 , 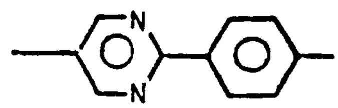 ,

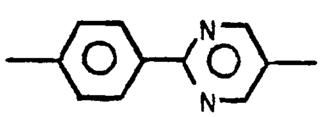 , 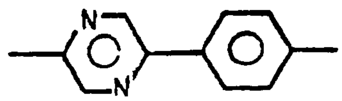 ,

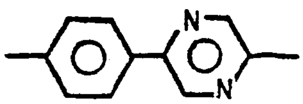 , 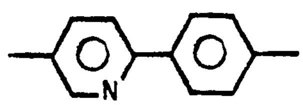 or 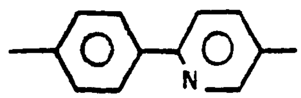

14. Optically active compound according to one of Claims 1 or 3 to 13, in which R° is methyl.

15. Optically active compound according to one of Claims 1 or 3 to 14, in which Y is a straight-chain alkyl group of up to 7 C atoms.

16. Optically active compound according to one of Claims 1 or 3 to 15, in which p is 1.

17. Optically active compound according to one of Claims 1 or 3 to 16, in which $X^1$ is —CO—O—.

18. Optically active compound according to one of Claims 1 or 3 to 16, in which $X^2$ is —O—CO—.

19. Liquid crystal phase containing at least one optically active compound of the formula I

$$R^1—X^1—CHR^\circ—(CH_2)_p—X^2—R^2 \qquad I$$

in which

$X^1$ and $X^2$, independently of one another, are each —CO—O— or —O—CO—; one of the groups $X^1$ and $X^2$ is also —O—, in which $R^1$ and $R^2$, independently of one another, are each a group $—(A^1—Z)_m—(A^2)_n—Y$, wherein

$A^1$ and $A^2$, in each case independently of one another [lacuna] a 1,4-phenylene, pyrimidine-2,5-diyl, pyrazine-2,5-diyl, pyridine-2,5-diyl, 1,4-cyclohexylene, 1,3-dioxane-2,5-diyl, 1,3-dithiane-2,5-diyl or 1,4-bicyclo-[2.2.2.]-octylene group, which can also be monosubstituted or polysubstituted by F, Cl, Br, CN and/or alkyl groups with up to 12 C atoms, and it being possible for 1 or 2 non-adjacent $CH_2$ groups in the alkyl groups to be replaced by O atoms,

Z is —CO—O—, —O—CO—, $—CH_2CH_2—$, $—OCH_2$, $—CH_2O—$, —CH=N—, —N=CH—, —N=N—, —N(O)=N— or a single bond,

m and n, in each case independently of one another [lacuna] each 0, 1 or 2, and

Y is a straight-chain or branched alkyl group of up to 12 C atoms, it being possible for 1 or 2 non-adjacent and non-terminal $CH_2$ groups to be replaced by O atoms, or, if n is 1 or 2, also F, Cl, Br or CN,

p is 0 or 1 and

R° is an alkyl group with up to 5 C atoms, a phenyl group or a cyclohexyl group.

20. Liquid crystal phase according to Claim 19, characterized in that it contains at least one optically active compound according to one of Claims 3 to 18.

21. Liquid crystal display elements containing a liquid crystal phase according to Claim 19.

22. Use of the compounds of the formula I according to Claim 1 for temperature compensation in liquid crystal phases.

23. Method of temperature compensation in liquid crytal display elements, characterized in that at least 0.05% of at least one compound of the formula I is admixed to the liquid crystal phase.